# EUROPEAN PATENT SPECIFICATION

(11) **EP 3 990 001 B1**
(45) Date of publication and mention of the grant of the patent: **04.11.2026**
(21) Application number: 20731873.4
(22) Date of filing: 12.06.2020
(51) Int. Cl.: A61K 38/17, A61K 38/28, A61P 3/10, A61P 5/50

(54) **COMBINATION OF S100 CALCIUM-BINDING PROTEIN A9 AND INSULIN IN THE TREATMENT OF TYPE 1 DIABETES**
KOMBINATION DES CALCIUM-BINDENDEN PROTEINS S100-A9 UND INSULIN BEI DER BEHANDLUNG VON TYP-1-DIABETES
COMBINAISON DE LA PROTÉINE LIANT LE CALCIUM S100-A9 ET DE L'INSULINE DANS LE TRAITEMENT DU DIABÈTE DE TYPE 1

(30) Priority: 28.06.2019 EP 19183317
(43) Date of publication of application: 04.05.2022
(73) Proprietor: Université de Genève, 1211 Genève (CH)
(72) Inventor: COPPARI, Roberto, 1206 Genève (CH); RAMADORI, Giorgio, 3014 Bern (CH); MIKROPOULOU, Despoina, 01280 Prevessin-Möens (FR)
(74) Representative: KATZAROV S.A.
(86) International application number: PCT/EP2020/066371
(87) International publication number: WO 2020/260043

(56) References cited:
- SIWEN WANG ET AL: "S100A8/A9 in Inflammation", FRONTIERS IN IMMUNOLOGY, vol. 9, 11 June 2018 (2018-06-11), XP055653073, DOI: 10.3389/fimmu.2018.01298
- FERNANDA DANA MANTELMACHER ET AL: "GIP regulates inflammation and body weight by restraining myeloid-cell-derived S100A8/A9", NATURE METABOLISM, vol. 1, no. 1, 19 November 2018 (2018-11-19), pages 58 - 69, XP055653095, DOI: 10.1038/s42255-018-0001-z
- MICHAEL J. KRAAKMAN ET AL: "Neutrophil-derived S100 calcium-binding proteins A8/A9 promote reticulated thrombocytosis and atherogenesis in diabetes", JOURNAL OF CLINICAL INVESTIGATION, vol. 127, no. 6, 1 June 2017 (2017-06-01), GB, pages 2133 - 2147, XP055652834, ISSN: 0021-9738, DOI: 10.1172/JCI92450
- FRANCISCO J. ORTEGA ET AL: "Targeting the association of calgranulin B (S100A9) with insulin resistance and type 2 diabetes", JOURNAL OF MOLECULAR MEDICINE., vol. 91, no. 4, 4 December 2012 (2012-12-04), DE, pages 523 - 534, XP055652767, ISSN: 0946-2716, DOI: 10.1007/s00109-012-0979-8
- GIORGIO RAMADORI ET AL: "S100A9 extends lifespan in insulin deficiency", NATURE COMMUNICATIONS, vol. 10, no. 1, 7 August 2019 (2019-08-07), XP055652761, DOI: 10.1038/s41467-019-11498-x
- LEUKERT N ET AL: "Calcium-dependent Tetramer Formation of S100A8 and S100A9 is Essential for Biological Activity", JOURNAL OF MOLECULAR BIOLOGY, ACADEMIC PRESS, UNITED KINGDOM, vol. 359, no. 4, 16 June 2006 (2006-06-16), pages 961 - 972, XP024951108, ISSN: 0022-2836, [retrieved on 20060616], DOI: 10.1016/J.JMB.2006.04.009

## Description

### CROSS REFERENCE TO RELATED APPLICATION

The present application claims the benefit of EP patent application serial number 19183317.7, filed on June 28, 2019.

### SEQUENCE LISTING

The Sequence Listing associated with this application is provided in text format in lieu of a paper copy. The name of the text file containing the Sequence Listing is PAT7278PC00_ST25.txt.

### FIELD OF THE INVENTION

The present disclosure provides compositions for their use in the treatment of type 1 diabetes, or an associated symptom, in a subject in need thereof, the compositions comprising a S100 calcium-binding protein A9 (S100A9), a variant or a fragment thereof and insulin, a variant or a fragment thereof.

### BACKGROUND OF THE INVENTION

Tens of millions suffer from type 1 diabetes (T1D); a condition caused by an autoimmune-mediated attack of pancreatic β-cells leading to total (or almost total) β-cell loss and insulin deficiency1. If untreated, T1D is a lethal catabolic disease characterized by hyperglycemia. Thus, the focus of T1D research and drug development has been mainly on improving strategies to lower hyperglycemia without causing life-threatening hypoglycemia ^{2,3}. However, in addition to increased circulating glucose level β-cell loss leads to several "other defects", some of which (e.g. severe hyperketonemia and ketoacidosis) are life-threatening⁴⁻⁷. Therefore, it is important to develop strategies that in addition to improve hyperglycemia can also rescue the "other defects" (e.g. increased ketogenesis) caused by insulin deficiency. For example, results shown below underscore the importance of ameliorating hyperglycemia and the "other defects". Indeed, our data indicate that despite the presence of slightly improved hyperglycemia a normalization of hyperketonemia and hypertriglyceridemia is associated with a significant extension in lifespan of mice with β-cell loss and insulin deficiency.

Untreated T1D rapidly leads to death ⁴. However, since insulin was discovered in the early 1920s ^{8,9}, T1D has been treated with insulin therapy; an approach that converted this lethal disease into one a person can live with. The remarkable achievement of insulin (which represents one of the most important discoveries in medicine) led to the conclusion that life without insulin is not possible, nevertheless the scientific community has to acknowledge that insulin therapy is unsatisfactory4. Indeed, T1D subjects have higher risks for developing kidney failure, blindness, nerve damage, heart attack, stroke, and hypoglycemia⁴. Some of these defects may be favored by insulin therapy itself. For example, insulin stimulates lipid and cholesterol synthesis; thus, probably owing to its established lipogenic actions¹⁰ chronic insulin therapy promotes lipid deposition outside adipose tissue. This effect could contribute to the extremely high incidence of coronary artery disease observed in diabetic subjects ^{5,6}. In addition, the lipogenic actions of insulin promote lipid-induced insulin resistance and therefore could underlie, at least in part, the increased insulin needs in long-term T1D care ¹¹. Insulin is also a potent glycemia-lowering hormone. Owing to this action, intensive insulin therapy causes hypoglycemia that can be disabling and sometimes could lead to death ¹²⁻¹⁴. Because insulin therapy does not eradicate the disabling co-morbidities of T1D (e.g. heart attack, stroke, blindness, kidney failure, neuropathy, etc.) the costs needed for T1D care are immense and the quality of life of T1D patients is reduced compared to normal subjects ¹⁵. Due to the shortcoming of current treatment, research aimed at improving T1D therapy is urgently needed.

The main approach is aiming at diminishing the amount of insulin dosage and hence reducing risks associated with insulin therapy as for example life-threatening hypoglycemia. Yet, virtually all the prospected adjunct treatments to insulin focus on improving hyperglycemia. For example, the synthetic analog of amylin (pramlintide), incretin mimetics (e.g. glucagon-like-peptide-1 receptor agonists and dipeptidyl-peptidase-4 inhibitors), and sodium-glucose-transporter-1 and -2 (SGLT1 and 2) inhibitors aim at lowering hyperglycemia and are associated with increased risks of hypoglycemia ^{2,3}. Some of these therapies are also associated with increased risks of ketoacidosis ^{2,3}.

Therefore, there remains a need for improved therapeutic methods for treating insulin deficiency (ID) condition, or an associated symptom, in a subject in need thereof which reduces the risks of hypoglycemia and ketoacidosis.

### SUMMARY OF THE INVENTION

The present invention provides a composition for use in the treatment of type 1 diabetes, or an associated symptom, in a subject in need thereof, the composition comprising
i) a S100 calcium-binding protein A9 (S100A9) having an amino-acid sequence as set forth in SEQ ID NO: 1, a biologically active variant sharing at least 90% sequence homology or a biologically active fragment thereof and
ii) insulin, a variant or a fragment thereof.

A further aspect of the invention concerns a plasmid or a vector for use in the treatment of type 1 diabetes, comprising one or more nucleic acid(s) encoding a S100 calcium-binding protein A9 (S100A9) having an amino-acid sequence as set forth in SEQ ID NO: 1, a biologically active variant sharing at least 90% sequence homology or a biologically active fragment thereof and insulin, a variant or a fragment thereof, and optionally an Affinity Tag.

A further aspect of the invention concerns one or more nucleic acid(s) encoding a S100 calcium-binding protein A9 (S100A9) having an amino-acid sequence as set forth in SEQ ID NO: 1 for use in the treatment of type 1 diabetes, a biologically active variant sharing at least 90% sequence homology or a biologically active fragment thereof and insulin, a variant or a fragment thereof, and optionally an Affinity Tag.

A further aspect of the invention concerns host cell for use in the treatment of type 1 diabetes comprising a plasmid or vector, or a nucleic acid of the invention.

A further aspect of the invention concerns a pharmaceutical composition for use in the treatment of type 1 diabetes comprising a therapeutically effective amount of i) a composition comprising a S100 calcium-binding protein A9 (S100A9) having an amino-acid sequence as set forth in SEQ ID NO: 1, a biologically active variant sharing at least 90% sequence homology or a biologically active fragment thereof and insulin, a variant or a fragment thereof, or ii) a plasmid or a vector of theinvention, or iii) a host cell of the invention, and at least one pharmaceutically acceptable excipient, diluent, carrier, salt and/or additive.

A further aspect of the invention concerns a protein or polypeptide comprising a S100A9 protein having an amino-acid sequence as set forth in SEQ ID NO: 1, a biologically active variant sharing at least 90% sequence homology or a biologically active fragment thereof, and insulin, a variant or a fragment thereof, and optionally, at least one affinity tag for use in the treatment of type 1 diabetes.

### DESCRIPTION OF THE FIGURES

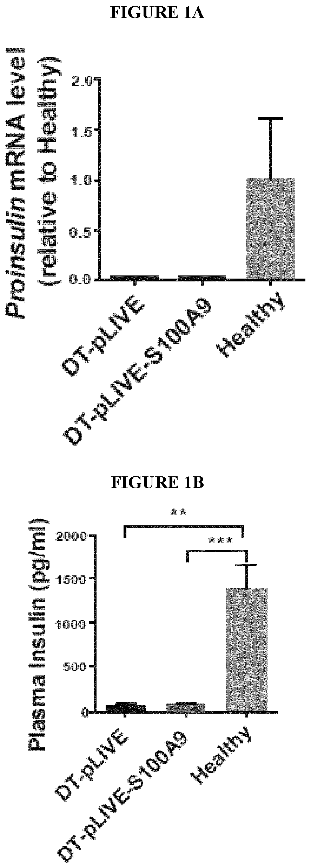

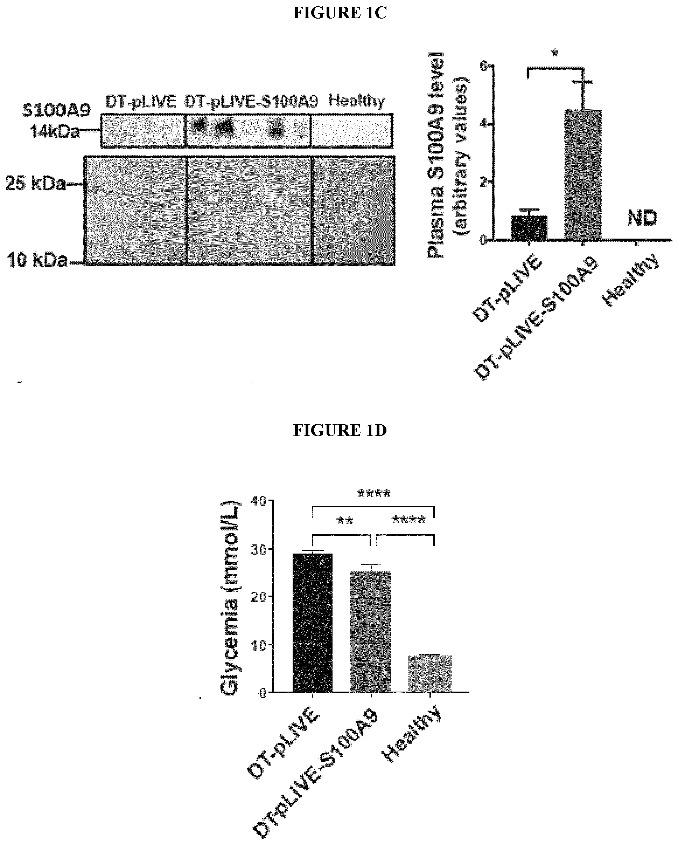

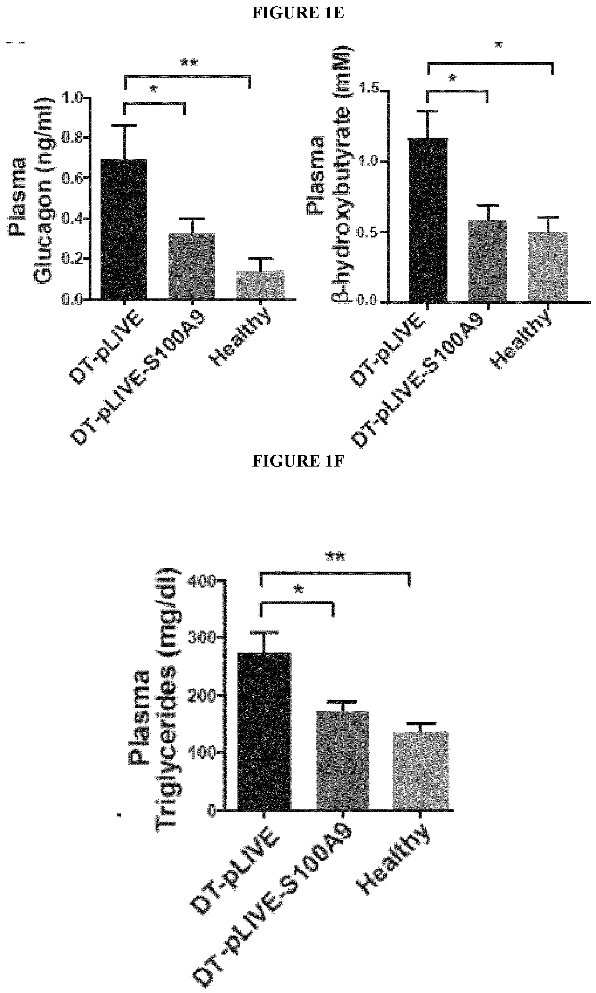

**Figure 1****. Murine S100A9 ameliorates metabolic imbalance in DT-induced ID mice. (a)** *Proinsulin* mRNA content in DT-treated *RIP-DTR* mice (sacrificed 10 days after hydrodynamic tail vein injection; HTVI) and their age-matched non-diabetic healthy controls. **(b)** Plasma insulin content in DT-pLIVE and DT-pLIVE-S100A9 mice (10 days after HTVI) and age-matched healthy controls. **(c)** Plasmatic S100A9 levels, and circulating **(d)** glucose, **(e)** glucagon and β-hydroxybutyrate, and **(f)** triglycerides. Error bars represent SEM. Statistical analyses were done using one-way ANOVA (Tukey's post-hoc test). Healthy (n = 3-6), DT-pLIVE (n = 7-12) and DT-pLIVE-S100A9 (n = 7-12). *P < 0.05; **P < 0.01; ***P < 0.001, ****P < 0.0001.

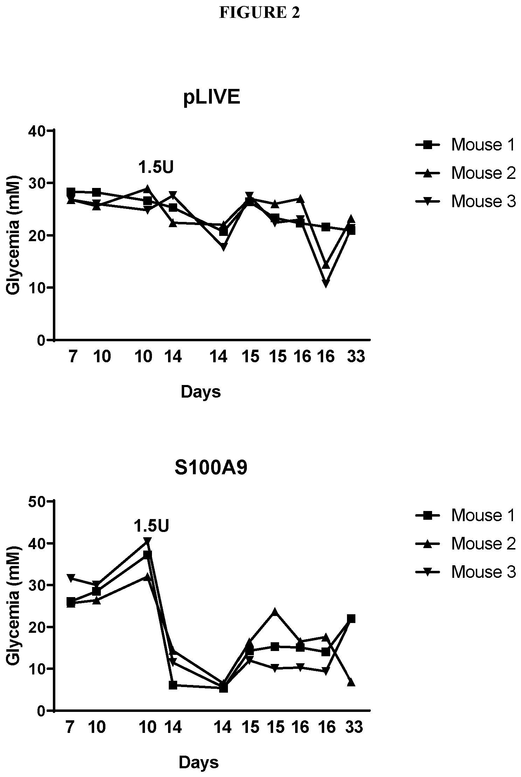

**Figure 2****. Enhanced murine S100A9 ameliorates insulin effectiveness in lowering hyperglycemia in ID mice.** The insulin dose at 1.5U/mouse did not affect hyperglycemia in DT-pLIVE mice (14 days after hydrodynamic tail vein injection; (HTVI)) while it did cause a rapid decrease in hyperglycemia in DT-pLIVE-S100A9 mice (14 days after HTVI). Glycemia was measured 3 hours after the insulin injection.

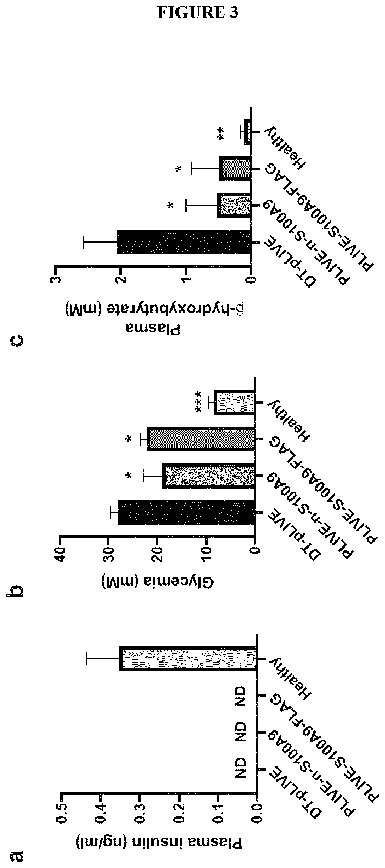

**Figure 3****. The presence of a C-terminal FLAG sequence does not interfere with the ability of murine S100A9 to improve ID symptoms in mice. (a)** Plasma insulin content in DT-pLIVE, DT-pLIVE-n-S 100A9 and DT-pLIVE-S100A9-FLAG mice (7 days after HTVI) and age-matched healthy controls (healthy) (ND = non-detectable). **(b)** Glycemia. **(c)** Plasma β-hydroxybutyrate level. For each group n= 4-9. Error bars represent SEM. Statistical analyses were done using one-way ANOVA (Tukey's post-hoc test). The statistical results represented for each condition are relative to DT-pLIVE group. *P < 0.05; **P < 0.01; ***P < 0.001

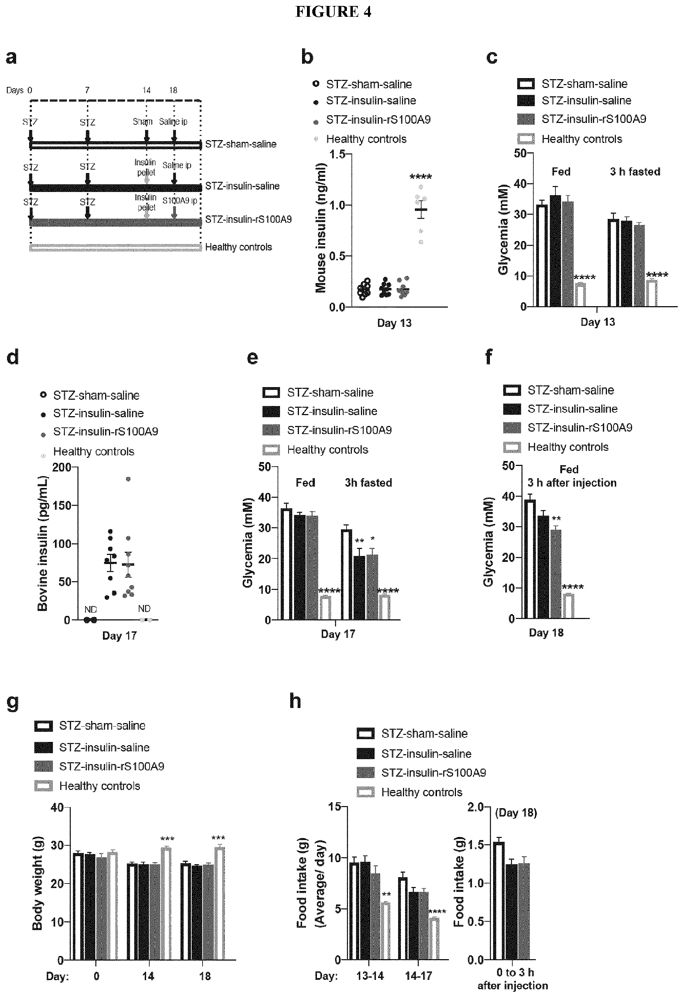

**Figure 4****. Recombinant murine S100A9 in combination with suboptimal insulin treatment ameliorates metabolic imbalance in ID mice. (A)** Experimental treatments and groups. **(B)** Plasma insulin levels and **(C)** glycaemia of mice 13 days after the first STZ injection (and of their healthy controls). **(D)** Plasma bovine insulin (released by the Linbit pellets) and **(E)** glycaemia of mice at the experimental day 17 (3 days after pellet implant) (and of their healthy controls). **(F)** Glycaemia of mice after intraperitoneal injection of rS100A9 or saline (and of their healthy controls). Injection of rS100A9 or saline was performed at Zeitgeber (ZT) 2. **(G)** Body weight. **(H)** Left panel represents daily or average/day food intake; Right panel represents food intake during 3 hours post-injection of either saline or rS100A9. Plasma values and glycaemia are from mice fed *ad libitum* ("fed") or after 3 hours of food removal ("3h fasted"), at the indicated experimental time. For each group n=8/9. In the insulin treated groups, all mice displaying plasma bovine insulin level inferior to 25 pg/mL were excluded from the study. In **C** and **E** fed and fasted values were obtained from plasma taken at ZT 2 and ZT 5, respectively. Error bars represent SEM. Statistical analyses were done using one-way ANOVA (Tukey's post-hoc test). The statistical results represented for each condition are relative to STZ-sham-saline group. *P < 0.05; **P < 0.01, ***P < 0.001, ****P < 0.0001.

### DESCRIPTION OF THE INVENTION

Although methods and materials similar or equivalent to those described herein can be used in the practice or testing of the present invention, suitable methods and materials are described below. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" or "comprising" is generally used in the sense of include/including, that is to say permitting the presence of one or more features or components. The terms "comprise" and "comprising" also encompass the more restricted ones "consist" and "consisting", respectively.

As used in the specification and claims, the singular form "a", "an" and "the" include plural references unless the context clearly dictates otherwise.

As used herein, "at least one" means "one or more", "two or more", "three or more", etc. For example, at least one affinity tag encompasses one, two or more, three or more, *etc...* affinity tag(s).

As used herein the terms "subject", "subject in need thereof", or "patient", "patient in need thereof " are well-recognized in the art, and, are used interchangeably herein to refer to a mammal, including dog, cat, rat, mouse, monkey, cow, horse, goat, sheep, pig, camel, and, most preferably, a human. In some cases, the subject is a subject in need of treatment or a subject with a disease or disorder. However, in other aspects, the subject can be a normal subject. The term does not denote a particular age or sex. Thus, adult and newborn subjects, whether male or female, are intended to be covered. Preferably, the subject is a human, most preferably a human suffering from insulin deficiency (ID) condition, or an associated symptom.

The term "treating", "treated" or "treatment" as used herein includes preventative (e.g. prophylactic), palliative, and curative uses or results.

The term "insulin deficiency" as used herein refers to a partial or complete loss of pancreatic insulin-producing beta-cells. The term further includes their reduced capacity of secreting insulin resulting in reduced level of circulating insulin.

The term "insulin deficiency associated symptom" as used herein refers to adverse effect(s) caused by low or absent levels of insulin.

Insulin deficiency associated symptom is selected from the group comprising hyperglycemia, hyperketonemia, ketoacidosis, hypertriglyceridemia, hyperglucagonemia, hypercalprotectinemia, increased or high circulating (non-esterified fatty acids (NEFAs) level, severehypoleptinemia, reduced or low body fat mass, hyperphagia, polydipsia and any combination thereof.

Insulin deficiency (ID) condition refers to diabetes type 1.

The terms "nucleic acid", "polynucleotide", and "oligonucleotide" are used interchangeably and refer to any kind of deoxyribonucleotide (e.g. DNA, cDNA, ...) or ribonucleotide (e.g. RNA, mPvNA, ...) polymer or a combination of deoxyribonucleotide and ribonucleotide (e.g. DNA/RNA) polymer, in linear or circular conformation, and in either single - or double - stranded form. These terms are not to be construed as limiting with respect to the length of a polymer and can encompass known analogues of natural nucleotides, as well as nucleotides that are modified in the base, sugar and/or phosphate moieties (e.g. phosphorothioate backbones). In general, an analogue of a particular nucleotide has the same base-pairing specificity; i.e., an analogue of A will base-pair with T.

The term "vector", as used herein, refers to a viral vector or to a nucleic acid (DNA or RNA) molecule such as a plasmid or other vehicle, which contains one or more heterologous nucleic acid sequence(s) (such as nucleic acid sequence(s) encoding the one or more nucleic acid(s) encoding the peptides (e.g. S100A9, Insulin, and/or Affinity Tag, variants or fragments thereof, of the invention). The terms "expression vector", "gene delivery vector" and "gene therapy vector" refer to any vector that is effective to incorporate and express one or more nucleic acid(s), in a cell, preferably under the regulation of a promoter (such as e.g. an inducible promoter as described in Kallunki T, Barisic M, Jäättelä M, Liu B. How to Choose the Right Inducible Gene Expression System for Mammalian Studies? *Cells.* 2019;8(8):796). A cloning or expression vector may comprise additional elements, for example, regulatory and/or post-transcriptional regulatory elements in addition to a promoter.

The term "about" particularly in reference to a given quantity, is meant to encompass deviations of plus or minus ten (10) percent (e.g. ± 10%). For example, about 20 consecutive amino-acids also encompasses 18 to 22 consecutive amino-acids, about 30 consecutive amino-acids also encompasses 27 to 33 consecutive amino-acids, *etc...*

As used herein, a "fragment" of a protein, peptide or polypeptide of the invention refers to a sequence containing less amino acids in length than the protein, peptide or polypeptide of the invention. This sequence can be used as long as it exhibits the same properties, i.e is biologically active, as the native sequence from which it derives.

The term "variant" refers to a protein, peptide or polypeptide having an amino acid sequence that differ to some extent from a native sequence peptide, that is an amino acid sequence that vary from the native sequence by amino acid substitutions, whereby one or more amino acids are substituted by another with same characteristics and conformational roles. The amino acid sequence variants possess substitutions, deletions, and/or insertions at certain positions within the amino acid sequence of the native amino acid sequence. Substitutions can also be conservative, in this case, the conservative amino acid substitutions are herein defined as exchanges within one of the following five groups:
I. Small aliphatic, nonpolar or slightly polar residues: Ala, Ser, Thr, Pro, Gly
II. Polar, positively charged residues: His, Arg, Lys
III. Polar, negatively charged residues: and their amides: Asp, Asn, Glu, Gin
IV. Large, aromatic residues: Phe, Tyr, Tip
V. Large, aliphatic, nonpolar residues: Met, Leu, Ile, Val, Cys.

The present invention is based, in part, on the surprising finding that the administration of S100 calcium-binding protein A9 (S100A9) along with an otherwise sub-optimal insulin dose greatly improves metabolism of insulin deficient mice without causing hypoglycaemia.

S100A9, that belongs to the EF-hand superfamily of Ca2+-binding proteins, is highly expressed in monocytes and neutrophils and secreted in conditions of elevated inflammation as for example in rheumatoid arthritis or sepsis16,17. With its partner S100A8, S100A9 forms a heterocomplex (S100A9/S100A8; also known as calprotectin) that is also secreted in response to inflammatory states 18. Calprotectin is an endogenous activator of the Toll-like receptor 4 (TLR4)17 and the receptor of advanced glycated end-products (RAGE)19. Calprotectin has been shown to exert several deleterious effects including underlying sepsis-induced lethality ^{16,17,19-21}. However, others have shown that calgranulins can also exist in monomers to exert anti-inflammatory effects20. Noteworthy, S100A9 homodimers have been reported to directly affect TLR4 signaling22. Collectively, these data indicate that calprotectin (S100A9/S100A8 heterodimer) and S100A9 (S100A9/S100A9 homodimer) regulate inflammatory pathways. Although calprotectin is considered to exert deleterious effects, there is murine and human evidence indicating that S100A9 is beneficial. For example, enhanced S100A9 brings about significant beneficial metabolic effects in T1D mice (Fig. 1).

An aspect of the present invention concerns a composition for use in the treatment of type 1 diabetes, or an associated symptom, in a subject in need thereof, the composition comprising a S100 calcium-binding protein A9 (S100A9) having an amino-acid sequence as set forth in SEQ ID NO: 1, a biologically active variant sharing at least 90% sequence homology or a biologically active fragment thereof and insulin, a variant or a fragment thereof.

The treatment comprises alleviating hyperglycemia, alleviating and/or reducing risk of hypoglycemia, alleviating increased level of glycated hemoglobin in the blood, alleviating hyperglucagonemia, alleviating and/or reducing risk of hyperketonemia and ketoacidosis, alleviating hypertriglyceridemia, alleviating increased hepatic fatty acid oxidation (FAO), increasing hepatic native or modified S 100A9 mRNA level, increasing hepatic native or modified S 100A9 protein level, increasing plasmatic native or modified S100A9 protein level, increasing hepatic ATP level, increasing lifespan, decreasing circulating non-esterified fatty acids (NEFAs) level, decreasing hepatic mitochondrial DNA level, decreasing circulating calprotectin level, decreasing lipase activity, or any combination thereof.

In certain aspects, the treatment comprises decreasing the insulin dose, or the variant or fragment thereof, by at least 5%, by at least 10%, by at least 15%, by at least 20%, by at least 25%, by at least 30%, by at least 35%, by at least 40%, by at least 45%, by at least 50%, or more as compared to the administration of insulin in the absence of a S 100A9 protein, a variant or a fragment thereof.

Preferably, the S100A9 protein is a native or recombinant protein having an amino-acid sequence as set forth in SEQ ID NO: 1, a biologically active variant sharing at least 90% sequence homology or a biologically active fragment thereof.

A fragment of the S 100A9 protein is preferably an active fragment comprising at least about 25 consecutive amino-acids, at least about 30 consecutive amino-acids, at least about 35 consecutive amino-acids, at least about 40 consecutive amino-acids, at least about 45 consecutive amino-acids, at least about 50 consecutive amino-acids, at least about 55 consecutive amino-acids, at least about 60 consecutive amino-acids, at least about 65 consecutive amino-acids, at least about 70 consecutive amino-acids, at least about 75 consecutive amino-acids, at least about 80 consecutive amino-acids, at least about 85 consecutive amino-acids, at least about 90 consecutive amino-acids, at least about 95 consecutive amino-acids, or at least about 100 consecutive amino-acids, at least about 105 consecutive amino-acids, or at least about 110 consecutive amino-acids, of the amino-acid sequence set forth in SEQ ID NO: 1.

Non-limiting examples of S100A9 fragments comprise S100A9 N91 (SEQ ID NO: 2), S100A9 C91 (SEQ ID NO: 3), S100A9 N76 (SEQ ID NO: 4) and S100A9 C76 (SEQ ID NO: 5), and a combination of one more thereof.

A variant of the S100A9 protein differs from the amino-acid sequence set forth in SEQ ID NO: 1, or from an active fragment thereof, in 1 to about 60 amino acids, preferably 1 to about 40 amino acids, more preferably 1 to about 20 amino acids, even more preferably 1 to about 10 amino acids. Preferably, the amino acid sequence variants are linear or cyclic peptides that possess substitutions, deletions at the N- and/or C-terminus, as well as within one or more internal domains, and/or insertions at certain positions within the amino acid sequence of the native amino acid sequence, or the SEQ ID No. 1, as described above.

The sequences of such variants are functionally, i.e. biologically, active variants and will have a high degree of sequence homology to the reference amino acid sequence, e.g., sequence homology of at least 90% or 95% or more, when the two sequences are aligned. This alignment and the percent homology or sequence identity can be determined using software programs known in the art, for example those described in Ausubel et al. eds. (2007) Current Protocols in Molecular Biology. Preferably, default parameters are used for alignment. One alignment program is BLAST, using default parameters. In particular, programs are BLASTN and BLASTP, using the following default parameters: Genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + SwissProtein + SPupdate + PIR. Biologically equivalent polynucleotides are those having the above-noted specified percent homology and encoding a polypeptide having the same or similar biological activity.

Non-limiting examples of S100A9 protein variants are selected from the group comprising SEQ ID No. 49, SEQ ID No. 50, SEQ ID No. 51, SEQ ID No. 52, and SEQ ID No. 53 and a combination of one more thereof. These sequences correspond to S100A9 proteins found in other animal species (e.g.) mammalian as shown in Table 1 below:

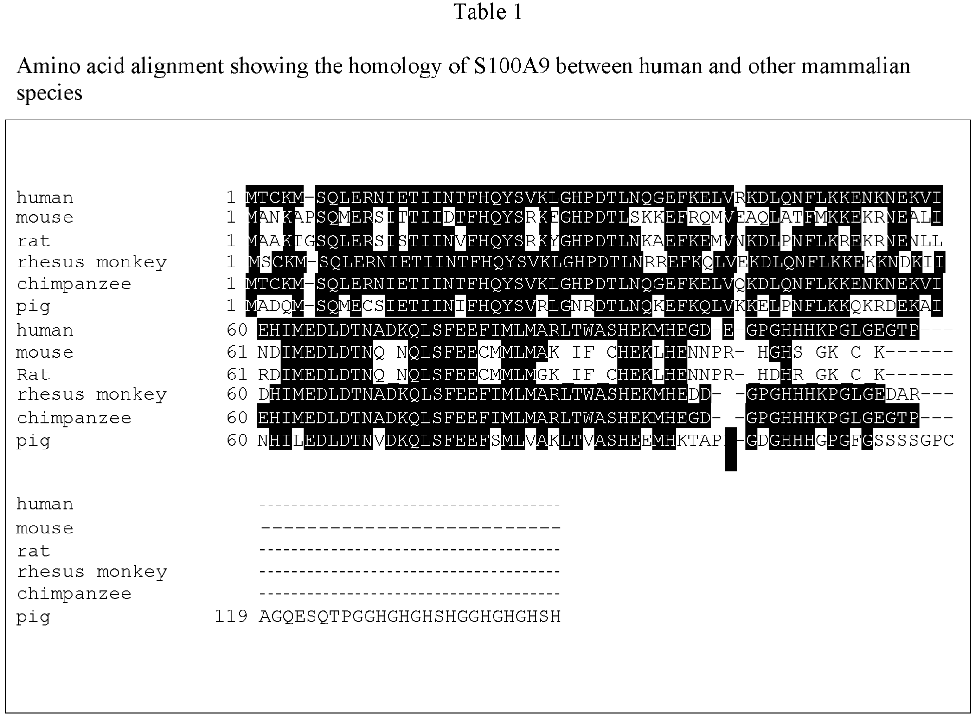

Both the variant and fragment of the S 100A9 protein can include synthetic, non-standard and/or naturally-occurring amino acid sequences (including D-forms and/or retro-inverso isomers) derivable from the naturally occurring amino acid sequence of the S100A9 protein. By way of example, the replacement amino acid may be a basic non-standard amino acid, (e.g. L-Ornithine, L-2-amino-3-guanidinopropionic acid, or D-isomers of Lysine, Arginine and Ornithine). Methods for introducing non-standard amino acids into proteins are known in the art, and include recombinant protein synthesis using E. coli auxotrophic expression hosts.

Non-naturally occurring amino acids include, without limitation, trans-3-methylproline, 2,4-methano-proline, cis-4-hydroxyproline, trans-4-hydroxy-proline, N-methylglycine, allo-threonine, methyl-threonine, hydroxy-ethylcysteine, hydroxyethylhomo-cysteine, nitro-glutamine, homoglutamine, pipecolic acid, tert-leucine, norvaline, 2-azaphenylalanine, 3-azaphenyl-alanine, 4-azaphenyl-alanine, and 4-fluorophenylalanine. Several methods are known in the art for incorporating non-naturally occurring amino acid residues into proteins.

A further example of an S100A9 variant comprises the S100A9 N69A-E78A variant having an amino-acid sequence as set forth in SEQ ID NO: 6.

The S 100A9 protein, variant or fragment thereof may also be conjugated to a chemical or enzymatic moiety. These moieties are typically used to increase solubility, prolong stability, reduce immunogenicity and/or enable fusion with an immunoglobulin or a particular region of an immunoglobulin. Non-limiting examples of these moieties comprise PEG, Maleimide-PEG(n)-succinimidyl ester and biotin.

Alternatively, the present invention also encompasses i) a S100A9 protein having an amino-acid sequence as set forth in SEQ ID NO: 1, a biologically active variant sharing at least 90% sequence homology or a biologically active fragment thereof, and insulin, a variant or a fragment thereof, and optionally, at least one affinity tag for use in the treatment of type 1 diabetes. When present, the at least one affinity tag is on the same peptide, in any order.

Insulin is selected from native insulin, recombinant insulin, proinsulin, basal insulin, insulin analogues, or bolus insulin. Insulin is a protein comprising a chain A and/or a chain B having an amino-acid sequence as set forth in, respectively SEQ ID NO: 7 and/or SEQ ID NO: 8, a variant or a fragment thereof.

A fragment of insulin protein refers to a fragment of chain A and/or chain B of insulin. Insulin fragments of A chain have an A chain length of at least about 15 consecutive amino-acids, at least about 16 consecutive amino-acids, at least about 17 consecutive amino-acids, at least about 18 consecutive amino-acids, at least about 19 consecutive amino-acids, at least about 20 consecutive amino-acids, at least about 21 consecutive amino-acids, at least about 22 consecutive amino-acids, at least about 23 consecutive amino-acids, at least about 24 consecutive amino-acids, at least about 25 consecutive amino-acids, at least about 26 consecutive amino-acids, at least about 27 consecutive amino-acids, at least about 28 consecutive amino-acids, at least about 29 consecutive amino-acids, at least about 30 consecutive amino-acids, at least about 35 consecutive amino-acids, or more of the native A chain amino acid sequence. Insulin fragments of B chain have a B chain length of at least about 25 consecutive amino-acids, at least about 26 consecutive amino-acids, at least about 27 consecutive amino-acids, at least about 28 consecutive amino-acids, at least about 29 consecutive amino-acids, at least about 29 consecutive amino-acids, at least about 30 consecutive amino-acids, at least about 31 consecutive amino-acids, at least about 32 consecutive amino-acids, at least about 33 consecutive amino-acids, at least about 34 consecutive amino-acids, at least about 35 consecutive amino-acids, at least about 36 consecutive amino-acids, at least about 37 consecutive amino-acids, at least about 38 consecutive amino-acids, at least about 39 consecutive amino-acids, at least about 40 consecutive amino-acids, at least about 41 consecutive amino-acids, at least about 42 consecutive amino-acids, at least about 42 consecutive amino-acids, at least about 43 consecutive amino-acids, at least about 44 consecutive amino-acids, at least about 45 consecutive amino-acids, at least about 50 consecutive amino-acids or more of the native amino acid B chain sequence.

A variant of the insulin protein is a linear or cyclic peptide that differs from the amino-acid sequences set forth in SEQ IDs NO: 7 and/or 8, or from an active fragment thereof, in 1 to about 60 amino acids, preferably 1 to about 40 amino acids, more preferably 1 to about 20 amino acids, even more preferably 1 to about 10 amino acids. Preferably, the amino acid sequence variants possess substitutions at the N- and/or C-terminus of at least one of the two chains, as well as within one or more internal domains, deletions, and/or insertions at certain positions within the amino acid sequence of the amino acid sequences as described above. The sequences of such variants are functionally, i.e. biologically, active variants and will have a high degree of sequence homologyto the reference amino acid sequence, e.g., sequence homology of more than 50%, generally more than 60%, even more particularly 80% or more, such as at least 90% or 95% or more, when the two sequences are aligned.

Non-limiting examples of insulin variants are selected from the group comprising insulin Lispro (SEQ IDs No. 9 and/or 10), insulin Glulisine (SEQ IDs No. 11 and/or 12), insulin Aspart (SEQ IDs No. 13 and/or 14), insulin Glargine (SEQ IDs No. 15 and/or 16), insulin Detemir (SEQ IDs No. 17 and/or 18), and insulin Deglutec (SEQ IDs No. 19 and/or 20), and a combination of one more thereof.

Both the variants and fragments of the insulin protein can include synthetic and/or naturally-occurring amino acid sequences (including D-forms and/or retro-inverso isomers) derivable from the naturally occurring amino acid sequence of the insulin protein and described above.

The insulin protein, variant or fragment thereof may also be conjugated to a chemical or enzymatic moiety. These moieties are typically used to increase solubility, prolong stability, reduce immunogenicity and/or enable fusion with an immunoglobulin or a particular region of an immunoglobulin. Non-limiting examples of these moieties comprise PEG and biotin. Examples can be found in, e.g., US 2010/0216690 and WO 2007/104738 (incorporated herein in their entirety).

A number of insulin analogues are known in the art. By way of examples, insulin analogues comprise those described e.g. in U.S. Pat. No. 5597796, EP1193272, US 20090069216 and WO 2007/096332 (incorporated herein in their entirety).

Proinsulin and proinsulin derivatives are also known in the art and can be selected, e.g. from those described in US20190263881.

In a certain aspect of the present invention, the S100A9 protein, variant or fragment thereof, further comprises at least one affinity tag. Said at least one affinity tag is attached to the C' and/or the N' terminus of the S100A9 protein, variant or fragment thereof

An affinity tag is usually fused to either the C' or N' terminus, or to both C' and N' terminuses, of a recombinant protein to facilitate affinity purification and detection. This approach enables high selective capture and circumvents the multistep purification processes that limit throughput during R&D.

The affinity tag of the invention can be any molecule, peptide or not, useful in both research and therapy that can be added to the S100A9 protein, variant or fragment thereof (Kimple et al., in Curr Protoc Protein Sci. ; 73: Unit-9.9., 2013) and/or to the insulin, variant or fragment thereof.

Preferably, the affinity tag is selected from the group comprising FLAG tag (SEQ ID NO: 21), chitin binding protein (CBP) tag (SEQ ID NO: 24), maltose binding protein (MBP) tag (SEQ ID NO: 25), Strep tag II (SEQ ID NO: 31), glutathione-S-transferase (GST) tag (SEQ ID NO: 32), poly(His) tag (SEQ ID NO: 33), C-myc (SEQ ID NO: 26), SBP (SEQ ID NO: 27), S (SEQ ID NO: 28), HAT (SEQ ID NO: 29), and a combination of one more thereof.

More preferably, the affinity tag is a FLAG tag consisting of, or comprising, the amino-acid sequence set forth in SEQ ID NO: 21 and a combination of one more thereof. Examples of combinations, or tandems, of the FLAG tag comprise 2x FLAG (SEQ ID NO: 22), 3x FLAG (SEQ ID NO: 23), etc...

Alternatively, the final tag of the 3x FLAG combination may encode an enterokinase cleavage site as set forth in SEQ ID NO: 23 (DYKDHD-G-DYKDHD-I-DYKDDDDK).

Non-limiting examples of S100A9 protein, variant or fragment thereof, that comprise one or more FLAG tag are selected from those listed in Table 2.

In a certain aspect of the invention, the i) S100A9 protein, variant or fragment thereof, ii) insulin, variant or fragment thereof, and, when present, the iii) at least one affinity tag are on the same peptide. Any combination can be envisioned, such as e.g. (from the N-terminus to the C-terminus): S100A9-Affinity Tag-Insulin, or S100A9- Insulin, or Insulin- S100A9, or Insulin - Affinity Tag-S100A9, or Affinity Tag-Insulin-S100A9, or Insulin-S100A9- Affinity Tag, or Affinity Tag-S100A9- Insulin, on the same peptide, separated or not by a peptidyl or non-peptidyl linker. An example of peptidyl linker (SEQ ID No. 47) is given in Table 2.

The compositions of the invention may further comprise a sodium-glucose cotransporter 1 (SGLT1) and/or 2 (SGLT2) inhibitor(s), amylin analogs, biguanides (e.g., metformin), incretin mimetics (e.g., glucagon-like peptide receptor agonists, dipeptidyl-peptidase-4 inhibitors).

The i) S100A9 protein, variant or fragment thereof of the invention and/or ii) insulin, variant or fragment thereof, optionally conjugated to an affinity tag, can be prepared by a variety of methods and techniques known in the art such as for example chemical synthesis or recombinant techniques as described in Maniatis et al. 1982, Molecular Cloning, A laboratory Manual, Cold Spring Harbor Laboratory.

The i) S100A9 protein, variant or fragment thereof of the invention and/or ii) insulin, variant or fragment thereof, optionally conjugated to an affinity tag, as described herein are preferably produced, recombinantly, in a cell expression system. A wide variety of unicellular host cells are useful in expressing the nucleic acid sequences encoding the peptides of the invention (e.g. S100A9, Insulin, and/or Affinity Tag), variants or fragments thereof. These hosts may include well known eukaryotic and prokaryotic hosts, such as strains of E. coli, Pseudomonas, Bacillus, Streptomyces, fungi such as yeasts, and animal cells, such as CHO, YB/20, NSO, SP2/0, Rl. 1, B-W and L-M cells, African Green Monkey kidney cells (e. g., COS 1, COS 7, BSCl, BSC40, and BMTIO), insect cells (e. g., Sf9), and human cells and plant cells in tissue culture.

The present invention also contemplates one or more nucleic acid(s) encoding the peptides of the invention (e.g. S100A9, Insulin, and/or Affinity Tag), variants or fragments thereof.

The present invention also contemplates a gene delivery vector, preferably in the form of a plasmid or a vector, for use in the treatment of type 1 diabetes, which comprises one or more nucleic acid(s) encoding the peptides of the invention (e.g. S100A9, Insulin, and/or Affinity Tag), variants or fragments thereof.

As used herein, a "vector" is capable of transferring nucleic acid sequences to target cells (e.g., viral vectors, non-viral vectors, particulate carriers, and liposomes).

Suitable vectors include derivatives of SV40 and known bacterial plasmids, e. g., E. coli plasmids col El, pCRl, pBR322, pLive, pMB9 and their derivatives, plasmids such as RP4; phage DNAs, e. g., the numerous derivatives of phage X, e. g., NM989, and other phage DNA, e. g., Ml 3 and filamentous single stranded phage DNA; yeast plasmids such as the 2µ plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like. Various viral vectors are used for delivering nucleic acid to cells in vitro or in vivo. Non-limiting examples are vectors based on Herpes Viruses, Pox- viruses, Adeno-associated virus, Lentivirus, and others. In principle, all of them are suited to deliver the expression cassette comprising an expressible nucleic acid molecule that codes for one or more nucleic acid(s) encoding the peptides (e.g. S100A9, Insulin, and/or Affinity Tag), variants or fragments thereof, of the invention.

In an aspect, said viral vector is a vector suited for ex-vivo and in-vivo gene delivery, More preferably, the viral vector is selected from the group comprising an adeno-associated virus (AAV) and a lentivirus, e.g. Lentivirus of 1st, 2nd, and 3rd generation, not excluding other viral vectors such as adenoviral vector, herpes virus vectors, etc .....Other means of delivery or vehicles are known (such as yeast systems, microvesicles, microemulsions, gene guns/means of attaching vectors to gold nanoparticles) and are provided, in some aspects, one or more of the viral or plasmid vectors may be delivered via liposomes, micro- or nanoparticles, exosomes, microvesicles, or a gene-gun.

In other aspects of the invention, the pharmaceutical composition(s) of the invention is/are a sustained-release formulation, or a formulation that is administered using a sustained-release device. Such devices are well known in the art, and include, for example, transdermal patches, and miniature implantable pumps that can provide for drug delivery over time in a continuous, steady-state fashionat a variety of doses to achieve a sustained-release effect with a non-sustained-release pharmaceutical composition.

Also contemplated in the present invention is a host cell for use in the treatment of type 1 diabetes comprising a gene delivery vector, preferably in the form of a plasmid or a vector, of the invention or one or more nucleic acid(s) encoding the peptides of the invention (e.g. S100A9, Insulin, and/or Affinity Tag), variants or fragments thereof. The host cell can be any prokaryotic or eukaryotic cell, preferably the host cell is a eukaryotic cell, most preferably the host cell is a mammalian cell. Even more preferably, the host cell is selected from the group comprising a pancreatic cell (e.g. β cell), an islet cell and a pancreatic precursor cell. Preferably, the host cell is a human β cell.

The invention further provides pharmaceutical compositions for use in the treatment of type 1 diabetes, comprising a therapeutically effective amount of a composition comprising i) a S100 calcium-binding protein A9 (S100A9) having an amino-acid sequence as set forth in SEQ ID NO: 1, a biologically active variant sharing at least 90% sequence homology or a biologically active fragment thereof and insulin, a variant or a fragment thereof, or ii) a plasmid or a vector of the invention, or iii) a host cell of the invention, and at least one pharmaceutically acceptable excipient, diluent, carrier, salt and/or additive.

The pharmaceutical compositions of the invention are for use in the treatment of type 1 diabetes in a subject in need thereof.

The term " therapeutically effective amount" as used herein means an amount of a composition, or peptide(s), of the invention high enough to significantly positively modify the symptoms and/or condition to be treated, but low enough to avoid serious side effects (at a reasonable risk/benefit ratio), within the scope of sound medical judgment.

The therapeutically effective amount of the composition, or peptide(s), of the invention is selected in accordance with a variety of factors including type, species, age, weight, sex and medical condition of the patient; the severity of the condition to be treated; the route of administration; the renal and hepatic function of the patient. A physician of ordinary skill in the art can readily determine and prescribe the effective amount of the drug required to prevent, counter or arrest the progress of an insulin deficiency (ID) condition, or an associated symptom.

Although therapeutically effective amount will vary from patient to patient, suitable daily amounts are in the range of about 0.1 to about 5000 mg (e.g., 0.1 , 0.5, 1 , 2, 5, 10, 15, 20, 25, 30, 35, 40, 45, 50, 75, 100, 150, 200, 250, 300, 350, 400, 450, 500, 550, 600, 650, 700, 750, 800, 850, 900, 950, 1000 mg, 1250 mg, 1500 mg, 1750 mg, 2000 mg, 2500 mg, 3000 mg, 3500 mg, 4000 mg, 4500 mg, 5000 mg, and the like, or any range or value therein) per patient, administered in single or multiple doses. Administration may be continuous or intermittent (e.g. by bolus injection). The dosage therapeutically effective amount also be determined by the timing and frequency of administration. In the case of oral or parenteral administration the dosage will preferably vary from about 1 mg to about 2000 mg per day of a peptide of the invention (or, if employed, a corresponding amount of a pharmaceutically acceptable salt or prodrug thereof). In particular aspects, the peptide of the invention, is administered to a subject at a daily dose in the range of from about 1 to about 2000 mg. The medical practitioner, or other skilled person, will be able to determine routinely the therapeutically effective amount which will be most suitable for an individual patient. The above-mentioned dosages are exemplary of the average case; there can, of course, be individual instances where higher or lower dosage ranges are merited, and such are within the scope of this invention. In some aspects, the pharmaceutical composition is administered as an injectable depot formulation.

"Pharmaceutically acceptable carrier or diluent" means a carrier or diluent that is useful in preparing a pharmaceutical composition that is generally safe, non-toxic, and desirable, and includes carriers or diluents that are acceptable for human pharmaceutical use.

Such pharmaceutically acceptable carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions.

Pharmaceutically acceptable excipients include starch, glucose, lactose, sucrose, sodium stearate, glycerol monostearate, talc, sodium chloride, dried skim milk, glycerol, propylene glycol, water, ethanol, phenol, protamine sulphate, zinc oxide and the like.

The pharmaceutical compositions may further contain one or more pharmaceutically acceptable salts such as, for example, a mineral acid salt such as a hydrochloride, a hydrobromide, a phosphate, a sulfate, etc.; and the salts of organic acids such as acetates, propionates, malonates, benzoates, etc. Additionally, auxiliary substances, such as wetting or emulsifying agents, pH buffering substances, gels or gelling materials, flavorings, colorants, microspheres, polymers, suspension agents, etc. may also be present herein. In addition, one or more other conventional pharmaceutical ingredients, such as preservatives, humectants, suspending agents, surfactants, antioxidants, anticaking agents, fillers, chelating agents, coating agents, chemical stabilizers, etc. may also be present, especially if the dosage form is a reconstitutable form. Suitable exemplary ingredients include macrocrystalline cellulose, carboxymethyf cellulose sodium, polysorbate 80, phenyletbyl alcohol, chiorobutanol, potassium sorbate, sorbic acid, sulfur dioxide, propyl gallate, the parabens, ethyl vanillin, glycerin, phenol, parachlorophenol, gelatin, albumin and a combination thereof. A thorough discussion of pharmaceutically acceptable excipients is available in REMINGTON'S PHARMACEUTICAL SCIENCES (Mack Pub. Co., N.J. 1991) which is incorporated by reference herein.

The pharmaceutical compositions of the present invention may be administered to a subject by different routes including orally, parenterally, sublingually, transdermally, rectally, transmucosally, topically, via inhalation, via buccal administration, intrapleurally, intravenous, intraarterial, intraperitoneal, subcutaneous, intramuscular, intranasal intrathecal, and intraarticular or combinations thereof. For human use, the composition may be administered as a suitably acceptable formulation in accordance with normal human practice. The skilled artisan will readily determine the dosing regimen and route of administration that is most appropriate for a particular patient. The compositions of the invention may be administered by traditional syringes, pumps, injection pens, micro-needle patches, indwelling catheter, needleless injection devices, "microprojectile bombardment gone guns", or other physical methods such as electroporation ("EP"), "hydrodynamic method", or ultrasound.

The pharmaceutical compositions of the present invention may also be delivered to the patient, by several technologies including DNA injection of nucleic acid encoding the peptides of the invention (e.g. S100A9, Insulin, and/or Affinity Tag) with and without in vivo electroporation, liposome mediated, nanoparticle facilitated, recombinant vectors such as recombinant lentivirus, recombinant adenovirus, and recombinant adenovirus associated virus as described herein.

The present invention also provide several technologies aimed at enhancing secretion of the peptides of the invention (e.g. S100A9, Insulin, and/or Affinity Tag) via implantation of i) wild-type and/or genetically engineered host cells, ii) drug-inducible engineered host cells, ii) light-inducible engineered host cells, iii) electromagnetically-inducible engineered host cells, and iv) electrically-inducible engineered host cells (see e.g. Krawczyk K, Xue S, Buchmann P, et al. Electrogenetic cellular insulin release for real-time glycemic control in type 1 diabetic mice. Science. 2020;368(6494):993-1001).

Also contemplated are several technologies aimed at enhancing the content of the peptides of the invention (e.g. S100A9, Insulin, and/or Affinity Tag) via implantation of reservoir materials (e.g. subcutaneous implanted solid pellets and/or hydrogels). Any combination of delivery methods and technologies disclosed herein are contemplated.

The invention also provides the use of compositions and pharmaceutical compositions of the invention in the preparation of a medicament for the treatment of type 1 diabetes,or an associated symptom.

The compositions or pharmaceutical compositions of the invention, are administered concomitantly, separately or staggered.

Combined or concomitant administration can include co-administration, either in a single pharmaceutical formulation or using separate formulations, or consecutive administration in either order but generally within a time period such that all active agents can exert their biological activities simultaneously. Preparation and dosing schedules for such agents can be used according to manufacturers' instructions or as determined empirically by the skilled practitioner.

**Table 2**

| **SEQ ID NO:** | **Name** | **Sequence** |
|---|---|---|
| 1 | S100A9 | MTCKMSQLERNIETIINTFHQYSVKLGHPDTLNQGEFKELVRKDLQN FLKKENKNEKVIEHIMEDLDTNADKQLSFEEFIMLMARLTWASHEK MHEGDEGPGHHHKPGLGEGTP |
| 2 | S100A9 N91 | MTCKMSQLERNIETIINTFHQYSVKLGHPDTLNQGEFKELVRKDLQN FLKKENKNEKVIEHIMEDLDTNADKQLSFEEFIMLMARLTWASH |
| 3 | S100A9 C91 | MKLGHPDTLNQGEFKELVRKDLQNFLKKENKNEKVIEHIMEDLDTN ADKQLSFEEFIMLMARLTWASHEKMHEGDEGPGHHHKPGLGEGTP |
| 4 | S100A9 N76 | MTCKMSQLERNIETIINTFHQYSVKLGHPDTLNQGEFKELVRKDLQN FLKKENKNEKVIEHIMEDLDTNADKQLSF |
| 5 | S100A9 C76 | MLVRKDLQNFLKKENKNEKVIEHIMEDLDTNADKQLSFEEFIMLMA RLTWASHEKMHEGDEGPGHHHKPGLGEGTP |
| 6 | S100A9 N69A E78A | MTCKMSQLERNIETIINTFHQYSVKLGHPDTLNQGEFKELVRKDLQN FLKKENKNEKVIEHIMEDLDTAADKQLSFEAFIMLMARLTWASHEK MHEGDEGPGHHHKPGLGEGTP |
| 7 | Insulin Human A chain | GIVEQCCTSICSLYQLENYCN |
| 8 | Insulin Human B chain | FVNQHLCGSHLVEALYLVCGERGFFYTPKT |
| 9 | Insulin Lispro A chain | GIVEQCCTSICSLYQLENYCN |
| 10 | Insulin Lispro B chain | FVNQHLCGSHLVEALYLVCGERGFFYTKPT |
| 11 | Insulin Glulisine A chain | GIVEQCCTSICSLYQLENYCN |
| 12 | Insulin Glulisine B chain | FVKQHLCGSHLVEALYLVCGERGFFYTPET |
| 13 | Insulin Aspart A chain | GIVEQCCTSICSLYQLENYCN |
| 14 | Insulin Aspart B chain | FVNQHLCGSHLVEALYLVCGERGFFYTDKT |
| 15 | Insulin Glargine A chain | GIVEQCCTSICSLYQLENYCG |
| 16 | Insulin Glargine B chain | FVNQHLCGSHLVEALYLVCGERGFFYTPKTRR |
| 17 | Insulin Detemir A chain | GIVEQCCTSICSLYQLENYCN |
| 18 | Insulin Detemir B chain | FVNQHLCGSHLVEALYLVCGERGFFYTPK myristic acid [CH3(CH2) 12COOH] is added to the epsilon-amino group of K in position 29 of chain B |
| 19 | Insulin Degludec A chain | GIVEQCCTSICSLYQLENYCN |
| 20 | Insulin Degludec B chain | FVNQHLCGSHLVEALYLVCGERGFFYTPK 16-carbon fatty acid is added to the epsilon-amino group of K in position 29 of chain B via a gamma-glutamic acid linker |
| 21 | 1xFLAG | DYKDDDDK |
| 22 | 2xFLAG | DYKDHD-G-DYKDHD |
| 23 | 3xFLAG | DYKDHD-G-DYKDHD-I-DYKDDDDK |
| 24 | CBP | TNPGVSAWQVNTAYTAGQLVTYNGKTYKCLQPHTSLAGWEPSNVP ALWQLQ |
| 25 | MBP | MKIEEGKLVIWINGDKGYNGLAEVGKKFEKDTGIKVTVEHPDKLEE KFPQVAATGDGPDIIFWAHDRFGGYAQSGLLAEITPDKAFQDKLYPF TWDAVRYNGKLIAYPIAVEALSLIYNKDLLPNPPKTWEEIPALDKEL KAKGKSALMFNLQEPYFTWPLIAADGGYAFKYENGKYDIKDVGVD NAGAKAGLTFLVDLIKNKHMNADTDYSIAEAAFNKGETAMTINGP WAWSNIDTSKVNYGVTVLPTFKGQPSKPFVGVLSAGINAASPNKEL AKEFLENYLLTDEGLEAVNKDKPLGAVALKSYEEELAKDPRIAATM ENAQKGEIMPNIPQMSAFWYAVRTAVINAASGRQTVDEALKDAQTN SSSNNNNNNNNNNLGIEGR |
| 26 | C-myc | EQKLISEEDL |
| 27 | SBP | MDEKTTGWRGGHVVEGLAGELEQLRARLEHHPQGQREP |
| 28 | S | KETAAAKFERQHMDS |
| 29 | HAT | KDHLIHNVHKEFHAHAHNK |
| 30 | Calmodulin binding peptide | KRRWKKNFIAVSAANRFKKISSSGAL |
| 31 | Strep-tag II | WSHPQFEK |
| 32 | GST | MSPILGYWKIKGLVQPTRLLLEYLEEKYEEHLYERDEGDK WRNKKFELGLEFPNLPYYIDGDVKLTQSMAIIRYIADKHN MLGGCPKERAEISMLEGAVLDIRYGVSRIAYSKDFETLKV DFLSKLPEMLKMFEDRLCHKTYLNGDHVTHPDFMLYDALD VVLYMDPMCLDAFPKLVCFKKRIEAIPQIDKYLKSSKYIA WPLQGWQATFGGGDHPPKSDLVPRGSPGIHRD |
| 33 | Poly-His | HHHHHH |
| 34 | Full S100A9 + FLAG | MTCKMSQLERNIETIINTFHQYSVKLGHPDTLNQGEFKELVRKDLQN FLKKENKNEKVIEHIMEDLDTNADKQLSFEEFIMLMARLTWASHEK MHEGDEGPGHHHKPGLGEGTPDYKDDDDK |
| 35 | FLAG + Full S100A9 | MDYKDDDDKMTCKMSQLERNIETIINTFHQYSVKLGHPDTLNQGEF KELVRKDLQNFLKKENKNEKVIEHIMEDLDTNADKQLSFEEFIMLM ARLTWASHEKMHEGDEGPGHHHKPGLGEGTP |
| 36 | S100A9 N91 Fragment + FLAG | MTCKMSQLERNIETIINTFHQYSVKLGHPDTLNQGEFKELVRKDLQN FLKKENKNEKVIEHIMEDLDTNADKQLSFEEFIMLMARLTWASHDY KDDDDK |
| 37 | FLAG + S100A9 N91 Fragment | MDYKDDDDKMTCKMSQLERNIETIINTFHQYSVKLGHPDTLNQGEF KELVRKDLQNFLKKENKNEKVIEHIMEDLDTNADKQLSFEEFIMLM ARLTWASH |
| 38 | S100A9 N76 Fragment + FLAG | MTCKMSQLERNIETIINTFHQYSVKLGHPDTLNQGEFKELVRKDLQN FLKKENKNEKVIEHIMEDLDTNADKQLSFDYKDDDDK |
| 39 | FLAG + S100A9 N76 Fragment | MDYKDDDDKMTCKMSQLERNIETIINTFHQYSVKLGHPDTLNQGEF KELVRKDLQNFLKKENKNEKVIEHIMEDLDTNADKQLSF |
| 40 | FLAG + S100A9 C91 Fragment | MDYKDDDDKMKLGHPDTLNQGEFKELVRKDLQNFLKKENKNEKVI EHIMEDLDTNADKQLSFEEFIMLMARLTWASHEKMHEGDEGPGHH HKPGLGEGTP |
| 41 | S100A9 C91 Fragment + FLAG | MKLGHPDTLNQGEFKELVRKDLQNFLKKENKNEKVIEHIMEDLDTN ADKQLSFEEFIMLMARLTWASHEKMHEGDEGPGHHHKPGLGEGTP DYKDDDDK |
| 42 | S100A9 C76 Fragment + FLAG | MLVRKDLQNFLKKENKNEKVIEHIMEDLDTNADKQLSFEEFIMLMA RLTWASHEKMHEGDEGPGHHHKPGLGEGTPDYKDDDDK |
| 43 | FLAG + S100A9 C76 Fragment | MDYKDDDDKMLVRKDLQNFLKKENKNEKVIEHIMEDLDTNADKQL SFEEFIMLMARLTWASHEKMHEGDEGPGHHHKPGLGEGTP |
| 44 | S100A9 N69A E78A + FLAG | MTCKMSQLERNIETIINTFHQYSVKLGHPDTLNQGEFKELVRKDLQN FLKKENKNEKVIEHIMEDLDTAADKQLSFEAFIMLMARLTWASHEK MHEGDEGPGHHHKPGLGEGTPDYKDDDDK |
| 45 | Seq. cloned into pLIVE between BamH1 and Xho1 sites | GCTAGCGGATCCGCCGCCACCATGGCCAACAAAGCACCTTCTCAG ATGGAGCGCAGCATAACCACCATCATCGACACCTTCCATCAATAC TCTAGGAAGGAAGGACACCCTGACACCCTGAGCAAGAAGGAATT CAGACAAATGGTGGAAGCACAGTTGGCAACCTTTATGAAGAAAG AGAAGAGAAATGAAGCCCTCATAAATGACATCATGGAGGACCTG GACACAAACCAGGACAATCAGCTGAGCTTTGAGGAGTGTATGAT GCTGATGGCAAAGTTGATCTTTGCCTGTCATGAGAAGCTGCATGA GAACAACCCACGTGGGCATGGCCACAGTCATGGCAAAGGCTGTG GGAAGGACTACAAAGACGATGACGACAAGTGACTCGAG |
| 46 | S100A9 C3S P114C | MTSKMSQLERNIETIINTFHQYSVKLGHPDTLNQGEFKELVRKDLQN FLKKENKNEKVIEHIMEDLDTNADKQLSFEEFIMLMARLTWASHEK MHEGDEGPGHHHKPGLGEGTC |
| 47 | Peptidyl linker | GSSGSSGSSGSSGSSG |
| 48 | Avi-Tag | GLNDIFEAQKIEWHE |
| 49 | Mouse | MANKAPSQMERSITTIIDTFHQYSRKEGHPDTLSKKEFRQMVEAQLA TFMKKEKRNEALINDIMEDLDTNQDNQLSFEECMMLMAKLIFACHE KLHENNPRGHGHSHGKGCGK |
| 50 | Rat | MAAKTGSQLERSISTIINVFHQYSRKYGHPDTLNKAEFKEMVNKDLP NFLKREKRNENLLRDIMEDLDTNQDNQLSFEECMMLMGKLIFACHE KLHENNPRGHDHRHGKGCGK |
| 51 | Rhesus monkey | MSCKMSQLERNIETIINTFHQYSVKLGHPDTLNRREFKQLVEKDLQN FLKKEKKNDKIIDHIMEDLDTNADKQLSFEEFIMLMARLTWASHEK MHEDDEGPGHHHKPGLGEDAR |
| 52 | chimpanzee | MTCKMSQLERNIETIINTFHQYSVKLGHPDTLNQGEFKELVQKDLQN FLKKENKNEKVIEHIMEDLDTNADKQLSFEEFIMLMARLTWASHEK MHEGDEGPGHHHKPGLGEGTP |
| 53 | pig | MADQMSQMECSIETIINIFHQYSVRLGNRDTLNQKEFKQLVKKELPN FLKKQKRDEKAINHILEDLDTNVDKQLSFEEFSMLVAKLTVASHEEM HKTAPPGDGHHHGPGFGSSSSGPCAGQESQTPGGHGHGHSHGGHGH GHSH |

The present invention also contemplates a delivery device comprising the pharmaceutical for use according to the invention. Preferably, the delivery device is selected from the group comprising a syringe injection, pump, pen, micro-needle patch, needle-free injection device, or indwelling catheter comprising the pharmaceutical composition for use of the invention.

While certain features of this invention have been illustrated and described herein, many modifications, substitutions, changes, and equivalents will now occur to those of ordinary skill in the art.

### REFERENCES

1. Wasserfall, C., et al. Persistence of Pancreatic Insulin mRNA Expression and Proinsulin Protein in Type 1 Diabetes Pancreata. Cell metabolism 26, 568-575 e563 (2017).
2. Harris, K., Boland, C., Meade, L. & Battise, D. Adjunctive therapy for glucose control in patients with type 1 diabetes. Diabetes Metab Syndr Obes 11, 159-173 (2018).
3. Lyons, S.K., et al. Use of Adjuvant Pharmacotherapy in Type 1 Diabetes: International Comparison of 49,996 Individuals in the Prospective Diabetes Follow-up and T1D Exchange Registries. Diabetes Care 40, e139-e140 (2017).
4. Coppari, R. & Bjorbaek, C. Leptin revisited: its mechanism of action and potential for treating diabetes. Nature reviews. Drug discovery 11, 692-708 (2012).
5. Larsen, J., et al. Silent coronary atheromatosis in type 1 diabetic patients and its relation to long-term glycemic control. Diabetes 51, 2637-2641 (2002).
6. Orchard, T.J., et al. Insulin resistance-related factors, but not glycemia, predict coronary artery disease in type 1 diabetes: 10-year follow-up data from the Pittsburgh Epidemiology of Diabetes Complications Study. Diabetes care 26, 1374-1379 (2003).
7. Umpierrez, G. & Korytkowski, M. Diabetic emergencies - ketoacidosis, hyperglycaemic hyperosmolar state and hypoglycaemia. Nature reviews. Endocrinology 12, 222-232 (2016).
8. Banting, F.G., Best, C.H., Collip, J.B., Campbell, W.R. & Fletcher, A.A. Pancreatic Extracts in the Treatment of Diabetes Mellitus. Canadian Medical Association journal 12, 141-146 (1922).
9. Banting, F.G., Campbell, W.R. & Fletcher, A.A. Further Clinical Experience with Insulin (Pancreatic Extracts) in the Treatment of Diabetes Mellitus. British medical journal 1, 8-12 (1923).
10. Horton, J.D., Goldstein, J.L. & Brown, M.S. SREBPs: activators of the complete program of cholesterol and fatty acid synthesis in the liver. The Journal of clinical investigation 109, 1125-1131 (2002).
11. Shulman, G.I. Cellular mechanisms of insulin resistance. The Journal of clinical investigation 106, 171-176 (2000).
12. Bulsara, M.K., Holman, C.D., Davis, E.A. & Jones, T.W. The impact of a decade of changing treatment on rates of severe hypoglycemia in a population-based cohort of children with type 1 diabetes. Diabetes Care 27, 2293-2298 (2004).
13. Cryer, P.E. Hypoglycemia-associated autonomic failure in diabetes. American journal of physiology. Endocrinology and metabolism 281, E1115-1121 (2001).
14. Cryer, P.E. Mechanisms of hypoglycemia-associated autonomic failure and its component syndromes in diabetes. Diabetes 54, 3592-3601 (2005).
15. Chiang, J.L., Kirkman, M.S., Laffel, L.M., Peters, A.L. & Type 1 Diabetes Sourcebook, A. Type 1 diabetes through the life span: a position statement of the American Diabetes Association. Diabetes Care 37, 2034-2054 (2014).
16. Averill, M.M., Kerkhoff, C. & Bornfeldt, K.E. S100A8 and S100A9 in cardiovascular biology and disease. Arteriosclerosis, thrombosis, and vascular biology 32, 223-229 (2012).
17. Loser, K., et al. The Toll-like receptor 4 ligands Mrp8 and Mrp14 are crucial in the development of autoreactive CD8+ T cells. Nature medicine 16, 713-717 (2010).
18. Leanderson, T., Liberg, D. & Ivars, F. S100A9 as a Pharmacological Target Molecule in Inflammation and Cancer. Endocrine, metabolic & immune disorders drug targets 15, 97-104 (2015).
19. Eggers, K., et al. RAGE-dependent regulation of calcium-binding proteins S100A8 and S100A9 in human THP-1. Experimental and clinical endocrinology & diabetes : official journal, German Society of Endocrinology [and] German Diabetes Association 119, 353-357 (2011).
20. Geczy, C.L., Chung, Y.M. & Hiroshima, Y. Calgranulins may contribute vascular protection in atherogenesis. Circulation journal : official journal of the Japanese Circulation Society 78, 271-280 (2014).
21. Vogl, T., et al. Mrp8 and Mrp14 are endogenous activators of Toll-like receptor 4, promoting lethal, endotoxin-induced shock. Nature medicine 13, 1042-1049 (2007).
22. Vogl, T., et al. Autoinhibitory regulation of S100A8/S100A9 alarmin activity locally restricts sterile inflammation. J Clin Invest 128, 1852-1866 (2018).
23. Blanco-Rojo, R., et al. Interaction of an S100A9 gene variant with saturated fat and carbohydrates to modulate insulin resistance in 3 populations of different ancestries. The American journal of clinical nutrition 104, 508-517 (2016).
24. Ortega, F.J., et al. Targeting the association of calgranulin B (S100A9) with insulin resistance and type 2 diabetes. J Mol Med (Berl) 91, 523-534 (2013).
25. Nyalwidhe, J.O., et al. Comparative quantitative proteomic analysis of disease stratified laser captured microdissected human islets identifies proteins and pathways potentially related to type 1 diabetes. PloS one 12, e0183908 (2017).
26. Zhi, W., et al. Discovery and validation of serum protein changes in type 1 diabetes patients using high throughput two dimensional liquid chromatography-mass spectrometry and immunoassays. Molecular & cellular proteomics : MCP 10, M111 012203 (2011).
27. Fujikawa, T., et al. Leptin engages a hypothalamic neurocircuitry to permit survival in the absence of insulin. Cell metabolism 18, 431-444 (2013).
28. Ramadori, G. et al. S100A9 extends lifespan in insulin deficiency. Nat Commun 10, 3545, doi:10.1038/s41467-019-11498-x (2019).
29. Fujikawa, T., Chuang, J. C., Sakata, I., Ramadori, G. & Coppari, R. Leptin therapy improves insulin-deficient type 1 diabetes by CNS-dependent mechanisms in mice. Proc Natl Acad Sci U S A 107, 17391-17396, doi:10.1073/pnas.1008025107 (2010).

### EXAMPLES

### Material & Methods

**Animals and induction of insulin-deficiency.** All mice were maintained with standard chow diet and water available *ad libitum* in a light- and temperature-controlled environment. All the experiments described in the study used adult male mice. Insulin deficient animal models were generated as follows: Diphtheria Toxin (DT, Sigma Aldrich) was dissolved in sterile 0.9% NaCl and intraperitoneally administrated into *RIP-DTR* animals (0.5 ug/kg body weight at days 0, 1, 4).

**Assessment of mRNA, protein, and substrates content.** Mice were sacrificed and their tissues quickly removed and snap-frozen in liquid nitrogen and subsequently stored at -80°C. RNAs were extracted using Trizol reagent (Invitrogen). Complementary DNA was generated by Superscript II (Invitrogen) and used with SYBR Green PCR master mix (Applied Biosystem, Foster City, CA, USA) for quantitative real time PCR (q-RTPCR) analysis. mRNA contents were normalized to *18s* mRNA levels. All assays were performed using an Applied Biosystems QuantStudio^{®} 5 Real-Time PCR System. For each mRNA assessment, q-RTPCR analyses were repeated at least 3 times. Proteins were extracted by homogenizing samples in lysis buffer (Tris 20mM, EDTA 5mM, NP40 1% (v/v), protease inhibitors (P2714-1BTL from Sigma, St. Louis, MO, USA), then resolved by SDS-PAGE and finally transferred to a nitrocellulose membrane by electroblotting. The following antibodies were used: CalgranulinB-S100A9 (cat. Number PB9678, Boster).

**Circulating substrates and hormones levels.** Tail vein blood was collected between 2 and 4 PM from mice that were fed *ad libitum.* To avoid random post-prandial confounding effects food was removed 2 hours prior to blood collection. Serum or plasma samples were collected after centrifugation (3500xg, 10 min) and stored at -80°C. Glucose, non-esterified fatty acids, triglycerides, ketone bodies, and glucagon levels were measured using commercially available kits.

**Overexpression of S100A9.** Hydrodynamic tail vein injection (HTVI) was performed. Overexpression of S100A9 was achieved by using pLIVE vectors (Myrus) that allow expression of a given gene under the control of the albumin promoter. The following sequences were cloned into pLIVE between restriction sites BamH1 and Xho1: 
GCTAGCGGATCCGCCGCCACCATGGCCAACAAAGCACCTTCTCAGATGGAGCGCAGCAT
AACCACCATCATCGACACCTTCCATCAATACTCTAGGAAGGAAGGACACCCTGACACCC
TGAGCAAGAAGGAATTCAGACAAATGGTGGAAGCACAGTTGGCAACCTTTATGAAGAA
AGAGAAGAGAAATGAAGCCCTCATAAATGACATCATGGAGGACCTGGACACAAACCAG
GACAATCAGCTGAGCTTTGAGGAGTGTATGATGCTGATGGCAAAGTTGATCTTTGCCTG
TCATGAGAAGCTGCATGAGAACAACCCACGTGGGCATGGCCACAGTCATGGCAAAGGC
TGTGGGAAGGACTACAAAGACGATGACGACAAGTGACTCGAG (SEQ ID No. 45).
 pLIVE-S100A9 plasmid DNA was sequenced to confirm correct sequences and orientation. Each mouse received 50 µg of pLIVE-S100A9 or pLIVE; aged-matched mice that did not undergo any procedure were used as healthy controls. Data shown in Fig. 2 were collected from mice injected intraperitoneally with 20 micro-grams of recombinant S100A9.

**Statistical analysis.** Data sets were analyzed for statistical significance using PRISM (GraphPad, San Diego, CA) for a two-tail unpaired Student's t test when two groups were compared or one-or two-way ANOVA (Tukey's post test) when more than two groups were compared.

### Example 1

### Results

### Beneficial metabolic actions of enhanced S100A9 in T1D mouse models

The Inventors overexpressed S100A9 in mice with insulin deficiency. The Inventors performed Hydrodynamic Tail Vein Injection studies in RIP-DTR mice that bear a rat insulin promoter (RIP) upstream of diphtheria toxin receptor (DTR) sequences cloned into the Hprt locus of the X chromosome. Following three consecutive intraperitoneal DT administrations, RIP-DTR mice develop a near-total-loss of pancreatic β-cells27. Indeed, almost all pancreatic β-cells were ablated in DT-injected RIP-DTR mice that underwent HTVI of either pLIVE (DT-pLIVE) or pLIVE-S100A9 (DT-pLIVE-S100A9) (data not shown). In line with β-cell loss, pancreatic Proinsulin mRNA level was barely measureable and these defects resulted in almost undetectable circulating insulin in DT-pLIVE and DT-pLIVE-S100A9 mice (Fig. 1a,b). To test whether DT-pLIVE-S100A9 mice have increased S100A9 we assessed plasmatic S100A9 level and found it to be increased in DT-pLIVE-S100A9 mice compared to DT-pLIVE and healthy controls (Fig. 1c). Collectively, these data demonstrate that DT-pLIVE-S100A9 mice are insulin deficient and overexpress S100A9. Owing to their ID, DT-pLIVE mice developed hyperglycemia, hyperketonemia, hypertriglyceridemia, and hyperglucagonemia (Figs. 1d-f). Next, the Inventors assessed the consequence of S100A9 overexpression on the aforementioned defects. Hyperglycemia was slightly improved in DT-pLIVE-S100A9 compared to DT-pLIVE mice (Fig. 1c). Remarkably, the circulating levels of glucagon, β-hydroxybutyrate, and triglycerides were all similar and significantly reduced between DT-pLIVE-S100A9 mice and healthy and DT-pLIVE controls, respectively (Fig. 1e-f).

### The therapeutic value of combination therapy between S100A9 and insulin in T1D

Results shown in Fig. 2 indicate a metabolic-improving and pro-survival action of enhanced S100A9 in T1D mice. The Inventors started testing whether enhanced S100A9 is able to reduce the insulin dose for management of T1D. Specifically, the Inventors increased circulating S100A9 level in combination with sub-optimal insulin dose (this is an insulin regimen that is not able to improve hyperglycemia and hyperketonemia caused by β-cell loss).

The results shown in Fig. 2 further indicate that while the sub-optimal dose of insulin did not affect hyperglycemia in control mice it significantly reduced hyperglycemia in mice overexpressing S100A9 without causing hypoglycemia.

### Example 2

**The presence of a C-terminal FLAG sequence does not interfere with the ability of S100A9 to improve ID symptoms in mice.**

To determine whether the addition of FLAG tag sequences to S100A9 can influence the ability of S100A9 to improve ID symptoms in mice we used HTVI to deliver either plasmid overexpressing full length native *S100a9* sequences (with or without a C-terminal FLAG tag) under the control of the albumin promoter (pLIVE-n-S100A9 or pLIVE-S100A9-FLAG) or the control empty vector (pLIVE). DT-treated *RIP-DTR* mice²⁷ that underwent HTVI of either pLIVE (DT-pLIVE) or pLIVE-n-S100A9 (DT-pLIVE-n-S100A9) or pLIVE-S100A9-FLAG (DT-pLIVE-S100A9-FLAG) showed a similar degree of hypoinsulinemia (Figure 3a). While DT-pLIVE mice showed hyperglycemia and hyperketonemia DT-pLIVE-n-S100A9 and DT-pLIVE-S100A9-FLAG mice displayed a similar improvement in these parameters (Figures 3b-c). These data demonstrate that the fusion of S100A9 with a FLAG tag sequence does not interfere the beneficial action of S100A9. Furthermore, these results indicate the possibility that, in addition to the FLAG sequence, other sequences of interest can be fused to S100A9 without interfering with its ability to improve ID symptoms.

**Recombinant S100A9 in combination with suboptimal insulin treatment ameliorates metabolic imbalance in ID mice.**

To directly test the therapeutic potential of S100A9 we assessed the metabolic outcome brought about by an injection of recombinant S100A9 (rS100A9) alone, or in combination with a suboptimal dose of insulin, in streptozotocin (STZ)-treated mice. As indicated in Figure 4a, 8-week-old male FVB mice were intraperitoneally (ip) injected with STZ (150mg/kg body weight) two times at one-week interval (this treatment generates an established model of ID)^{27, 28, 29}. Two weeks after the first STZ injection mice were randomized in three groups: i) the "STZ-insulin-rS100A9" group was surgically implanted with a subcutaneous insulin pellet (consisting in a half of a Linbit pellet, Linshin-Canada, expected to continuously deliver bovine insulin at a dose causing minimal effect on glycaemia) and three days after surgery these mice were ip injected with rS100A9 (1 mg/kg body weight); ii) the "STZ-insulin-saline" group was surgically implanted with a subcutaneous insulin pellet as indicated above and three days after surgery these mice were ip injected with saline; iii) the "STZ-sham-saline" group was surgically treated as the previous group and three days after surgery these mice were ip injected with saline. Age-matched-untreated mice were included as healthy controls (Figure 4a). As expected, STZ treatment led to severe insulinopenia and hyperglycemia (Figures 4b-c). Three days after surgery, STZ-insulin-rS100A9 and STZ-insulin-saline mice exhibited detectable plasma level of bovine insulin (Figure 4d) while bovine insulin was expectedly not measurable in STZ-sham-saline, and healthy mice (Figure 4d). In line with the suboptimal insulin dosage, three days after surgery STZ-insulin-rS100A9 and STZ-insulin-saline mice were hyperglycemic as they presented only a modest reduction of glycaemia after 3 hours of food removal compared to the STZ-sham-saline group (Figure 4e). Next, we monitored the acute metabolic effects of ip injected rS100A9.

Our data show that three hours after injection the hyperglycemia was comparable between STZ-insulin-saline and STZ-sham-insulin mice (Figure 4f). However, combination of insulin and ip injection of rS100A9 was able to cause a small but significant decrease in glycaemia in the STZ-insulin-rS100A9 group compared to the STZ-sham-insulin group (Figures 4f). Of note, these data were obtained from ID mice of similar body weight. Indeed, STZ treatment caused a similar reduction of body weight in all three groups while either insulin or rS100A9 treatment, or the combination of both, did not affect body weight (Figure 4g). Moreover, all the three ID groups showed hyperphagia with a trend toward a reduction of food intake in mice treated with insulin (and with no additional effect caused by rS100A9 injection) (Figure 4h). Overall, these data demonstrate that an ip injection of rS100A9 (1 mg/kg body weight) in combination with a suboptimal dose of insulin, is sufficient to bring about beneficial effects on glycemia in ID mice.

## Claims

1. A composition for use in the treatment of type 1 diabetes, or an associated symptom, in a subject in need thereof, the composition comprising
i) a S100 calcium-binding protein A9 (S100A9) having an amino-acid sequence as set forth in SEQ ID NO: 1, a biologically active variant sharing at least 90% sequence homology or a biologically active fragment thereof and
ii) insulin, a variant or a fragment thereof.

2. The composition for use of claim 1, wherein the treatment comprises decreasing the insulin dose, or the variant or fragment thereof, by at least 5%, by at least 10%, by at least 15%, by at least 20%, by at least 25%, by at least 30%, by at least 35%, by at least 40%, by at least 45%, by at least 50%, or more as compared to the administration of insulin in the absence of the S100A9 protein, a variant or a fragment thereof.

3. The composition for use of claim 1 or 2, wherein i) the amino-acid sequence of the S100A9 protein variant differs from the amino-acid sequence set forth in SEQ ID NO: 1, or from an active fragment thereof, in 1 to about 10 amino acids and wherein ii) the fragment of the S100A9 protein is an active fragment comprising at least about 25 consecutive amino-acids, at least about 30 consecutive amino-acids, at least about 35 consecutive amino-acids, at least about 40 consecutive amino-acids, at least about 45 consecutive amino-acids, at least about 50 consecutive amino-acids, at least about 55 consecutive amino-acids, at least about 60 consecutive amino-acids, at least about 65 consecutive amino-acids, at least about 70 consecutive amino-acids, at least about 75 consecutive amino-acids, at least about 80 consecutive amino-acids, at least about 85 consecutive amino-acids, at least about 90 consecutive amino-acids, at least about 95 consecutive amino-acids, or at least about 100 consecutive amino-acids, at least about 105 consecutive amino-acids, or at least about 110 consecutive amino-acids, of the amino-acid sequence set forth in SEQ ID NO: 1.

4. The composition for use of any one of claims 1 to 3, wherein insulin is native insulin, proinsulin, basal insulin or bolus insulin.

5. The composition for use of any one of the preceding claims, wherein the amino-acid sequence of the insulin protein is as set forth in SEQ ID NO: 7 and/or SEQ ID NO: 8.

6. The composition for use of any one of the preceding claims, wherein i) the amino-acid sequence of the insulin protein variant differs from the amino-acid sequence set forth in SEQ ID NO: 7 and/or SEQ ID NO: 8, or from an active fragment thereof, in 1 to about 60 amino acids, preferably 1 to about 40 amino acids, more preferably 1 to about 20 amino acids, even more preferably 1 to about 10 amino acids, and ii) wherein the insulin variant is selected from the group comprising insulin Lispro (SEQ IDs No. 9 and/or 10), insulin Glulisine (SEQ IDs No. 11 and/or 12), insulin Aspart (SEQ IDs No. 13 and/or 14), insulin Glargine (SEQ IDs No. 15 and/or 16), insulin Detemir (SEQ IDs No. 17 and/or 18), and insulin Deglutec (SEQ IDs No. 19 and/or 20), and a combination of one more thereof.

7. The composition for use of any one of the preceding claims, wherein type 1 diabetes associated symptom is selected from the group comprising hyperglycemia, hyperketonemia, ketoacidosis, hypertriglyceridemia, hyperglucagonemia, increased or high circulating non-esterified fatty acids (NEFAs) level, hyperphagia, polydipsia and any combination thereof.

8. The composition for use of any one of the preceding claims, wherein the treatment comprises alleviating hyperglycemia, alleviating and/or reducing risk of hypoglycemia, alleviating increased level of glycated hemoglobin in the blood, alleviating hyperglucagonemia, alleviating and/or reducing risk of hyperketonemia and ketoacidosis, alleviating hypertriglyceridemia, alleviating increased hepatic fatty acid oxidation (FAO), increasing hepatic native or modified S 100A9 mRNA level, increasing hepatic native or modified S 100A9 protein level, increasing plasmatic native or modified S 100A9 protein level, decreasing circulating non-esterified fatty acids (NEFAs) level, decreasing lipase activity, or any combination thereof.

9. The composition for use of any one of the preceding claims, wherein the i) S100A9 protein, variant or fragment thereof, and ii) the insulin, variant or fragment thereof, are administered concomitantly, separately or staggered.

10. The composition for use of any one of the preceding claims further comprising a sodium-glucose cotransporter 1 (SGLT1) and/or 2 (SGLT2) inhibitor(s), amylin analogs, biguanides (e.g., metformin), incretin mimetics (e.g., glucagon-like peptide receptor agonists, dipeptidyl-peptidase-4 inhibitors).

11. A plasmid or a vector for use in the treatment of type 1 diabetes, comprising one or more nucleic acid(s) encoding a S100 calcium-binding protein A9 (S100A9) having an amino-acid sequence as set forth in SEQ ID NO: 1, a biologically active variant sharing at least 90% sequence homology or a biologically active fragment thereof and insulin, a variant or a fragment thereof, and optionally an Affinity Tag.

12. One or more nucleic acid(s) encoding a S100 calcium-binding protein A9 (S100A9) having an amino-acid sequence as set forth in SEQ ID NO: 1 for use in the treatment of type 1 diabetes, a biologically active variant sharing at least 90% sequence homology or a biologically active fragment thereof and insulin, a variant or a fragment thereof, and optionally an Affinity Tag.

13. A host cell for use in the treatment of type 1 diabetes comprising a plasmid or vector of claim 11, or a nucleic acid of claim 12.

14. A pharmaceutical composition for use in the treatment of type 1 diabetes comprising a therapeutically effective amount of i) a composition comprising a S100 calcium-binding protein A9 (S100A9) having an amino-acid sequence as set forth in SEQ ID NO: 1, a biologically active variant sharing at least 90% sequence homology or a biologically active fragment thereof and insulin, a variant or a fragment thereof, or ii) a plasmid or a vector of claim 11, or iii) a host cell of claim 13,
and at least one pharmaceutically acceptable excipient, diluent, carrier, salt and/or additive.

15. A protein or polypeptide comprising
a S100A9 protein having an amino-acid sequence as set forth in SEQ ID NO: 1, a biologically active variant sharing at least 90% sequence homology or a biologically active fragment thereof, and insulin, a variant or a fragment thereof, and optionally, at least one affinity tag for use in the treatment of type 1 diabetes.

16. A pharmaceutical composition for use according to claim 14, wherein the pharmaceutical composition is comprised in a delivery device selected from the group comprising a syringe injection, pump, pen, micro-needle patch, needle-free injection device, or indwelling catheter.

## Patentansprüche

1. Zusammensetzung zur Verwendung bei der Behandlung von Typ-1-Diabetes oder einem damit assoziierten Symptom bei einem Probanden mit Bedarf daran, wobei die Zusammensetzung Folgendes umfasst:
i) ein S100-Calcium-bindendes Protein A9 (S100A9) mit einer Aminosäuresequenz, wie in SEQ ID NO: 1 dargelegt, eine biologisch aktive Variante, die mindestens 90 % Sequenzhomologie aufweist, oder ein biologisch aktives Fragment davon und
ii) Insulin, eine Variante oder ein Fragment davon.

2. Zusammensetzung zur Verwendung nach Anspruch 1, wobei die Behandlung Verringern der Dosis an Insulin oder der Variante oder des Fragments davon um mindestens 5 %, mindestens 10 %, mindestens 15 %, mindestens 20 %, mindestens 25 %, mindestens 30 %, mindestens 35 %, mindestens 40 %, mindestens 45 %, mindestens 50 % oder mehr im Vergleich zur Verabreichung von Insulin in Abwesenheit des S100A9-Proteins, einer Variante oder eines Fragments davon umfasst.

3. Zusammensetzung zur Verwendung nach Anspruch 1 oder 2, wobei i) sich die Aminosäuresequenz der Variante des S100A9-Proteins von der in SEQ ID NO: 1 dargelegten Aminosäuresequenz oder von einem aktiven Fragment davon um 1 bis etwa 10 Aminosäuren unterscheidet und wobei ii) das Fragment des S100A9-Proteins ein aktives Fragment ist, das mindestens etwa 25 aufeinanderfolgende Aminosäuren, mindestens etwa 30 aufeinanderfolgende Aminosäuren, mindestens etwa 35 aufeinanderfolgende Aminosäuren, mindestens etwa 40 aufeinanderfolgende Aminosäuren, mindestens etwa 45 aufeinanderfolgende Aminosäuren, mindestens etwa 50 aufeinanderfolgende Aminosäuren, mindestens etwa 55 aufeinanderfolgende Aminosäuren, mindestens etwa 60 aufeinanderfolgende Aminosäuren, mindestens etwa 65 aufeinanderfolgende Aminosäuren, mindestens etwa 70 aufeinanderfolgende Aminosäuren, mindestens etwa 75 aufeinanderfolgende Aminosäuren, mindestens etwa 80 aufeinanderfolgende Aminosäuren, mindestens etwa 85 aufeinanderfolgende Aminosäuren, mindestens etwa 90 aufeinanderfolgende Aminosäuren, mindestens etwa 95 aufeinanderfolgende Aminosäuren oder mindestens etwa 100 aufeinanderfolgende Aminosäuren, mindestens etwa 105 aufeinanderfolgende Aminosäuren oder mindestens etwa 110 aufeinanderfolgende Aminosäuren der die in SEQ ID NO: 1 dargelegten Aminosäuresequenz umfasst.

4. Zusammensetzung nach einem der Ansprüche 1 bis 3, wobei es sich bei dem Insulin um natives Insulin, Proinsulin, Basalinsulin oder Bolusinsulin handelt.

5. Zusammensetzung nach einem der vorhergehenden Ansprüche, wobei die Aminosäuresequenz des Insulinproteins wie in SEQ ID NO: 7 und/oder SEQ ID NO: 8 dargelegt ist.

6. Zusammensetzung zur Verwendung nach einem der vorhergehenden Ansprüche, wobei i) sich die Aminosäuresequenz der Variante des Insulinproteins von der SEQ ID NO: 7 und/oder SEQ ID NO: 8 dargelegten Aminosäuresequenz oder von einem aktiven Fragment davon um 1 bis etwa 60 Aminosäuren, vorzugsweise 1 bis etwa 40 Aminosäuren, bevorzugter 1 bis etwa 20 Aminosäuren, noch bevorzugter 1 bis etwa 10 Aminosäuren unterscheidet, und ii) wobei die Insulinvariante ausgewählt ist aus der Gruppe, die Insulin-Lispro (SEQ-ID-Nr. 9 und/oder 10), Insulin-Glulisin (SEQ-ID-Nr. 11 und/oder 12), Insulin-Aspart (SEQ-ID-Nr. 13 und/oder 14), Insulin-Glargin (SEQ-ID-Nr. 15 und/oder 16), Insulin-Detemir (SEQ-ID-Nr. 17 und/oder 18) und Insulin-Degludec (SEQ-ID-Nr. 19 und/oder 20) sowie eine Kombination aus einem oder mehreren davon umfasst.

7. Zusammensetzung zur Verwendung nach einem der vorhergehenden Ansprüche, wobei das mit Typ-1-Diabetes assoziierte Symptom ausgewählt ist aus der Gruppe, die Hyperglykämie, Hyperketonämie, Ketoazidose, Hypertriglyceridämie, Hyperglukagonämie, einen erhöhten oder hohen Spiegel von zirkulierenden (nicht veresterten) Fettsäuren (NEFA), Hyperphagie, Polydipsie und eine Kombination davon umfasst.

8. Zusammensetzung zur Verwendung nach einem der vorhergehenden Ansprüche, wobei die Behandlung Lindern von Hyperglykämie, Lindern und/oder Verringern des Risikos von Hypoglykämie, Lindern eines erhöhten Spiegels von glykiertem Hämoglobin im Blut; Lindern von Hyperglukagonämie, Lindern und/oder Verringern des Risikos von Hyperketonämie und Ketoazidose, Lindern von Hypertriglyceridämie, Lindern einer erhöhten hepatischen Fettsäureoxidation (FAO), Erhöhen des Leberspiegels von nativer oder modifizierter S100A9-mRNA, Erhöhen des Leberspiegels von nativem oder modifiziertem S100A9-Protein, Erhöhen des Plasmaspiegels von nativem oder modifiziertem S100A9-Protein, Verringern des Spiegels von zirkulierenden nicht veresterten Fettsäuren (NEFA), Verringern der Lipaseaktivität oder eine beliebige Kombination davon umfasst.

9. Zusammensetzung zur Verwendung nach einem der vorhergehenden Ansprüche, wobei das i) S100A9-Protein, die Variante oder das Fragment davon und ii) das Insulin, die Variante oder das Fragment davon gleichzeitig, getrennt oder zeitlich versetzt verabreicht werden.

10. Zusammensetzung zur Verwendung nach einem der vorhergehenden Ansprüche, ferner umfassend einen oder mehrere Natrium-Glukose-Cotransporter-1-(SGLT1)- und/oder - 2-(SGLT2)-Inhibitoren, Amylin-Analoga, Biguanide (z. B. Metformin), Inkretin-Mimetika (z. B. Glucagon-ähnliche Peptid-Rezeptor-Agonisten, Dipeptidylpeptidase-4-Inhibitoren).

11. Plasmid oder Vektor zur Verwendung bei der Behandlung von Typ-1-Diabetes, umfassend eine oder mehrere Nukleinsäure(n), die für ein S100-Calcium-bindendes Protein A9 (S100A9) mit einer in SEQ ID NO: 1 dargelegten Aminosäuresequenz, eine biologisch aktive Variante, die mindestens 90 % Sequenzhomologie aufweist, oder ein biologisch aktives Fragment davon codieren, und Insulin, eine Variante oder ein Fragment davon und gegebenenfalls ein Affinity-Tag.

12. Eine oder mehrere Nukleinsäuren, die für ein S100-Calcium-bindendes Protein A9 (S100A9) mit einer in SEQ ID NO: 1 dargelegten Aminosäuresequenz zur Verwendung bei der Behandlung von Typ-1-Diabetes, eine biologisch aktive Variante, die mindestens 90 % Sequenzhomologie aufweist, oder ein biologisch aktives Fragment davon codieren, und Insulin, eine Variante oder ein Fragment davon und gegebenenfalls ein Affinity-Tag.

13. Wirtszelle zur Verwendung bei der Behandlung von Typ-1-Diabetes, umfassend ein Plasmid oder einen Vektor nach Anspruch 11 oder eine Nukleinsäure nach Anspruch 12.

14. Pharmazeutische Zusammensetzung zur Verwendung bei der Behandlung von Typ-1-Diabetes, umfassend eine therapeutisch wirksame Menge i) einer Zusammensetzung, die ein S100-Calcium-bindendes Protein A9 (S100A9) mit einer in SEQ ID NO: 1 dargelegten Aminosäuresequenz, eine biologisch aktive Variante, die mindestens 90 % Sequenzhomologie aufweist, oder ein biologisch aktives Fragment davon, und Insulin, eine Variante oder ein Fragment davon umfasst, oder ii) eines Plasmids oder eines Vektors nach Anspruch 11 oder iii) einer Wirtszelle nach Anspruch 13,
und mindestens einen pharmazeutisch verträglichen Hilfsstoff, ein pharmazeutisch verträgliches Verdünnungsmittel, einen pharmazeutisch verträglichen Träger, ein pharmazeutisch verträgliches Salz und/oder einen pharmazeutisch verträglichen Zusatzstoff.

15. Protein oder Polypeptid, umfassend
ein S100A9-Protein mit einer Aminosäuresequenz, wie dargelegt in SEQ ID NO: 1, eine biologisch aktive Variante, die mindestens 90 % Sequenzhomologie aufweist, oder ein biologisch aktives Fragment davon und Insulin, eine Variante oder ein Fragment davon und gegebenenfalls ein Affinity-Tag zur Verwendung bei der Behandlung von Typ-1-Diabetes.

16. Pharmazeutische Zusammensetzung zur Verwendung nach Anspruch 14, wobei die pharmazeutische Zusammensetzung in einer Verabreichungsvorrichtung umfasst ist, die aus der Gruppe ausgewählt ist, die eine Injektionsspritze, eine Pumpe, einen Pen, ein Mikronadelpflaster, eine nadelfreie Injektionsvorrichtung oder einen Verweilkatheter umfasst.

## Revendications

1. Composition destinée à être utilisée dans le traitement du diabète de type 1, ou d'un symptôme associé, chez un sujet en ayant besoin, la composition comprenant
i) une protéine A9 de liaison au calcium S100 (S100A9) ayant une séquence d'acides aminés telle que présentée dans SEQ ID NO : 1, un variant biologiquement actif partageant une homologie de séquence d'au moins 90% ou un fragment biologiquement actif de celle-ci, et
ii) de l'insuline, un variant ou un fragment de celle-ci.

2. Composition destinée à être utilisée selon la revendication 1, dans laquelle le traitement comprend une diminution de la dose d'insuline, ou du variant ou du fragment de celle-ci, d'au moins 5%, d'au moins 10%, d'au moins 15%, d'au moins 20%, d'au moins 25%, d'au moins 30%, d'au moins 35%, d'au moins 40%, d'au moins 45%, d'au moins 50%, ou plus par rapport à l'administration d'insuline en l'absence de la protéine S100A9, d'un variant ou d'un fragment de celle-ci.

3. Composition destinée à être utilisée selon la revendication 1 ou 2, dans laquelle i) la séquence d'acides aminés du variant de la protéine S100A9 diffère de la séquence d'acides aminés présentée dans SEQ ID NO : 1, ou d'un fragment actif de celle-ci, en 1 à environ 10 acides aminés, et dans laquelle ii) le fragment de la protéine S100A9 est un fragment actif comprenant au moins environ 25 acides aminés consécutifs, au moins environ 30 acides aminés consécutifs, au moins environ 35 acides aminés consécutifs, au moins environ 40 acides aminés consécutifs, au moins environ 45 acides aminés consécutifs, au moins environ 50 acides aminés consécutifs, au moins environ 55 acides aminés consécutifs, au moins environ 60 acides aminés consécutifs, au moins environ 65 acides aminés consécutifs, au moins environ 70 acides aminés consécutifs, au moins environ 75 acides aminés consécutifs, au moins environ 80 acides aminés consécutifs, au moins environ 85 acides aminés consécutifs, au moins environ 90 acides aminés consécutifs, au moins environ 95 acides aminés consécutifs, ou au moins environ 100 acides aminés consécutifs, au moins environ 105 acides aminés consécutifs, ou au moins environ 110 acides aminés consécutifs, de la séquence d'acides aminés présentée dans SEQ ID NO : 1.

4. Composition destinée à être utilisée selon l'une quelconque des revendications 1 à 3, dans laquelle l'insuline est de l'insuline native, de la proinsuline, de l'insuline basale ou de l'insuline en bolus.

5. Composition destinée à être utilisée selon l'une quelconque des revendications précédentes, dans laquelle la séquence d'acides aminés de la protéine d'insuline est telle que présentée dans SEQ ID NO : 7 et/ou SEQ ID NO : 8.

6. Composition destinée à être utilisée selon l'une quelconque des revendications précédentes, dans laquelle i) la séquence d'acides aminés du variant de la protéine d'insuline diffère de la séquence d'acides aminés présentée dans SEQ ID NO : 7 et/ou SEQ ID NO : 8, ou d'un fragment actif de celle-ci, en 1 à environ 60 acides aminés, de préférence 1 à environ 40 acides aminés, plus préférablement 1 à environ 20 acides aminés, encore plus préférablement 1 à environ 10 acides aminés, et ii) dans laquelle le variant d'insuline est choisi dans le groupe comprenant l'insuline Lispro (SEQ ID No : 9 et/ou 10), l'insuline Glulisine (SEQ ID No : 11 et/ou 12), l'insuline Asparte (SEQ ID No : 13 et/ou 14), l'insuline Glargine (SEQ ID No : 15 et/ou 16), l'insuline Détémir (SEQ ID No : 17 et/ou 18) et l'insuline Dégludec (SEQ ID No : 19 et/ou 20) et une combinaison d'une ou de plusieurs de celles-ci.

7. Composition destinée à être utilisée selon l'une quelconque des revendications précédentes, dans laquelle le symptôme associé au diabète de type 1 est choisi dans le groupe comprenant l'hyperglycémie, l'hypercétonémie, l'acidocétose, l'hypertriglycéridémie, l'hyperglucagonémie, un niveau d'acides gras non estérifiés (AGNE) circulant accru ou élevé, l'hyperphagie, la polydipsie et toute combinaison de ceux-ci.

8. Composition destinée à être utilisée selon l'une quelconque des revendications précédentes, dans laquelle le traitement comprend l'atténuation de l'hyperglycémie, l'atténuation et/ou la réduction du risque d'hypoglycémie, l'atténuation de l'augmentation du niveau d'hémoglobine glyquée dans le sang, l'atténuation de l'hyperglucagonémie, l'atténuation et/ou la réduction du risque d'hypercétonémie et d'acidocétose, l'atténuation de l'hypertriglycéridémie, l'atténuation de l'augmentation de l'oxydation des acides gras hépatiques (FAO), l'augmentation du niveau d'ARNm de S100A9 hépatique natif ou modifié, l'augmentation du niveau de protéine S100A9 hépatique native ou modifiée, l'augmentation du niveau de protéine S100A9 plasmatique native ou modifiée, la diminution du niveau d'acides gras non estérifiés (AGNE) circulant, la diminution de l'activité lipase, ou toute combinaison de celles-ci.

9. Composition destinée à être utilisée selon l'une quelconque des revendications précédentes, dans laquelle i) la protéine S100A9, son variant ou son fragment, et ii) l'insuline, son variant ou son fragment, sont administrés de manière concomitante, séparément ou de manière échelonnée.

10. Composition destinée à être utilisée selon l'une quelconque des revendications précédentes, comprenant en outre un/des inhibiteur(s) de cotransporteur sodium-glucose 1 (SGLT1) et/ou 2 (SGLT2), des analogues de l'amyline, des biguanides (par exemple, la metformine), des mimétiques de l'incrétine (par exemple, des agonistes de récepteur de peptide de type glucagon, des inhibiteurs de la dipeptidyl-peptidase-4).

11. Plasmide ou vecteur destiné à être utilisé dans le traitement du diabète de type 1, comprenant un ou plusieurs acide(s) nucléique(s) codant pour une protéine A9 de liaison au calcium S100 (S100A9) ayant une séquence d'acides aminés telle que présentée dans SEQ ID NO : 1, un variant biologiquement actif partageant une homologie de séquence d'au moins 90% ou un fragment biologiquement actif de celle-ci, et de l'insuline, un variant ou un fragment de celle-ci, et éventuellement un marqueur d'affinité.

12. Acide(s) nucléique(s) codant pour une protéine A9 de liaison au calcium S100 (S100A9) ayant une séquence d'acides aminés telle que présentée dans SEQ ID NO : 1, pour une utilisation dans le traitement du diabète de type 1, un variant biologiquement actif partageant une homologie de séquence d'au moins 90% ou un fragment biologiquement actif de celle-ci et de l'insuline, un variant ou un fragment de celle-ci, et éventuellement un marqueur d'affinité.

13. Cellule hôte destinée à être utilisée dans le traitement du diabète de type 1 comprenant un plasmide ou un vecteur selon la revendication 11, ou un acide nucléique selon la revendication 12.

14. Composition pharmaceutique destinée à être utilisée dans le traitement du diabète de type 1 comprenant une quantité thérapeutiquement efficace i) d'une composition comprenant une protéine A9 de liaison au calcium S100 (S100A9) ayant une séquence d'acides aminés telle que présentée dans SEQ ID NO : 1, un variant biologiquement actif partageant une homologie de séquence d'au moins 90% ou un fragment biologiquement actif de celle-ci et de l'insuline, un variant ou un fragment de celle-ci, ou ii) d'un plasmide ou d'un vecteur selon la revendication 11, ou iii) d'une cellule hôte selon la revendication 13,
et au moins un excipient, diluant, support, sel et/ou additif pharmaceutiquement acceptable(s).

15. Protéine ou polypeptide comprenant
une protéine S100A9 ayant une séquence d'acides aminés telle que présentée dans SEQ ID NO : 1, un variant biologiquement actif partageant une homologie de séquence d'au moins 90% ou un fragment biologiquement actif de celle-ci, et de l'insuline, un variant ou un fragment de celle-ci, et éventuellement au moins un marqueur d'affinité pour une utilisation dans le traitement du diabète de type 1.

16. Composition pharmaceutique destinée à être utilisée selon la revendication 14, dans laquelle la composition pharmaceutique est comprise dans un dispositif d'administration choisi dans le groupe comprenant une seringue d'injection, une pompe, un stylo, un timbre à micro-aiguilles, un dispositif d'injection sans aiguille ou un cathéter à demeure.
